# EUROPEAN PATENT APPLICATION

(11) **EP 4 831 188 A1**
(43) Date of publication of application: **28.10.2026**
(21) Application number: 24911283.0
(22) Date of filing: 26.12.2024
(51) Int. Cl.: C07J 63/00, C07J 71/00, A61K 31/56, A61K 31/58, A61P 29/00, A61P 37/00, A61P 1/00, A61P 13/12, A61P 11/00

(54) **GLYCYRRHETINIC ACID DERIVATIVE AND USE THEREOF**

(30) Priority: 27.12.2023 CN 202311821957
(71) Applicant: Tianjin Pharmaceutical Biotechnology Co., Ltd, Tianjin 300171 (CN)
(72) Inventor: ZHANG, Yanqiao, Tianjin 300171 (CN); SUN, Shuangyong, Tianjin 300171 (CN); ZHAO, Xinying, Tianjin 300171 (CN); CHENG, Chunhui, Tianjin 300171 (CN); ZHANG, Jiaojiao, Tianjin 300171 (CN); YU, Yue, Tianjin 300171 (CN); YUAN, Yi, Tianjin 300171 (CN); SUN, Xiaomei, Tianjin 300171 (CN); ZHANG, Jie, Tianjin 300171 (CN); HAN, Kunying, Tianjin 300171 (CN); LIU, Dengke, Tianjin 300171 (CN)
(74) Representative: Bayramoglu et al.
(86) International application number: PCT/CN2024/142568
(87) International publication number: WO 2025/140361

(57) **Abstract**

Provided in the present disclosure is a glycyrrhetinic acid derivative as shown in formula (A) or a pharmaceutically acceptable salt thereof. The compound of the present disclosure can be used in the treatment of inflammatory diseases or autoimmune diseases.

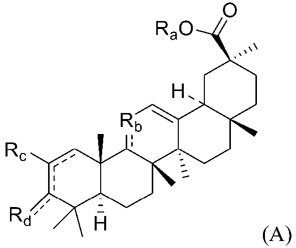

## Description

### Technical Field

The present disclosure is in the field of medical technology, and in particular, relates to a glycyrrhetinic acid derivative and use thereof, including, but not limited to a use in the treatment of dermatitis, eczema, atopic dermatitis, seborrhoeic dermatitis, hormone-dependent dermatitis, photosensitive dermatitis, chloasma, psoriasis, urticaria, prurigo nodularis, pityriasis rosea, inflammatory bowel disease, lupus erythematosus, alopecia areata, pemphigus, bullous pemphigoid, chronic nephritis, asthma, rhinitis, skin lesions caused by radiotherapy, burns, or scalds.

### Background Technology

Glycyrrhetinic acid is a triterpenoid saponin component of glycyrrhizic acid, a natural product derived from the traditional Chinese medicine *Glycyrrhiza uralensis Fisch.* (licorice), which is the main pharmacologically active component of licorice. Glycyrrhetinic acid belongs to a pentacyclic triterpenoid, with a C=O group at position 11 of its skeleton, resembling the 18-H-oleanane type structure. Glycyrrhetinic acid exists as different optical isomers: 18α-form and 18β-form (as shown in the figure below).

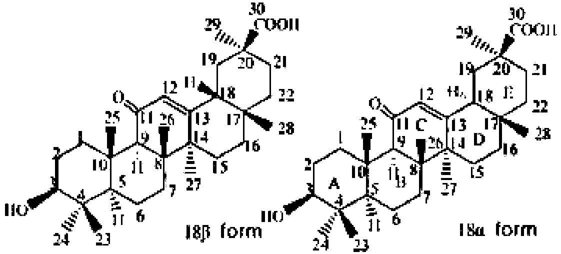

Studies have shown that glycyrrhetinic acid and derivatives thereof have a wide range of pharmacological activities, including anti-inflammatory, anti-ulcer, antiviral, hypolipidemic, free radical-scavenging, anti-tumor, and other biological activities. In terms of anti-inflammation, glycyrrhetinic acid and derivatives thereof can directly inhibit the production of inflammatory cytokines and inflammatory mediators through multiple targets, and also indirectly exert their anti-inflammatory effects by mechanisms such as enhancing the activity of steroid hormones. However, the biological activities of glycyrrhetinic acid are generally weak, and its anti-inflammatory activity is approximately 1/10 that of hydrocortisone, which limits its clinical application. Therefore, there is an urgent need for the development of compounds with favorable biological activities.

In view of the above, the present disclosure is hereby proposed.

### Content of the Invention

The main objective of the present disclosure is to provide a glycyrrhetinic acid derivative and use thereof, with a view to at least partially addressing at least one of the above technical problems.

In general, provided in the present disclosure is a compound as shown in formula (A) or a pharmaceutically acceptable salt thereof: 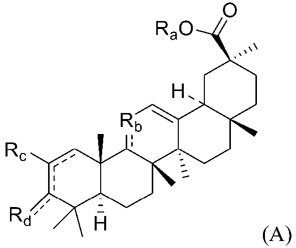
wherein Rₐ is -(CH₂)ₙ-NR₂₁R₂₂, where R₂₁ and R₂₂ may be the same or different and are each independently selected from hydrogen and C1-C6 alkyl, or R₂₁ and R₂₂, together with the nitrogen atom to which they are attached, form a 5- to 6-membered heterocyclic group, which heterocyclic group may optionally contain one or more additional heteroatoms selected from O, N, or NH, and
n = an integer from 1 to 6;
R_{c} is selected from hydrogen, halogen, hydroxyl, cyano, methoxy, amino, or haloalkyl;
R_{b} is selected from carbonyl or methine;
-̅ -̅ -̅ -̅ is a single bond or a double bond;
R_{d} is oxo;
----- represents that the H atom attached to the C atom is in the α-configuration or β-configuration; or,
Rₐ is hydrogen; R_{b} is carbonyl; R_{c} represents Br or Cl; R₁ is oxo; -̅ -̅ -̅ -̅ is a single bond or a double bond; and ----- represents that the H atom attached to the C atom is in the α-configuration or β-configuration;
   or
Rₐ is selected from hydrogen or -(CH₂)ₙ-NR₂₁R₂₂, where R₂₁ and R₂₂ may be the same or different and are each independently selected from hydrogen and C1-C6 alkyl, or R₂₁ and R₂₂, together with the nitrogen atom to which they are attached, form a 5- to 6-membered heterocyclic group, which heterocyclic group may optionally contain one or more additional heteroatoms selected from O, N, or NH, and n = an integer from 1 to 6;
R_{b} is carbonyl;
R_{c} and R_{d} are each independently selected from N, O, or S, and, together with the carbon atom to which they are attached, form a 5- to 6-membered aromatic heterocyclic group, which aromatic heterocyclic group is optionally substituted with NR₁₁R₁₂, where R₁₁ and R₁₂ are each independently selected from C1-C6 alkyl;
-̅ -̅ -̅ -̅ is a single bond or a double bond;
----- represents that the H atom attached to the C atom is in the α-configuration or β-configuration; provided that the compound as shown in formula (A) is not 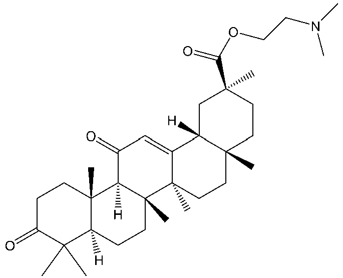 or

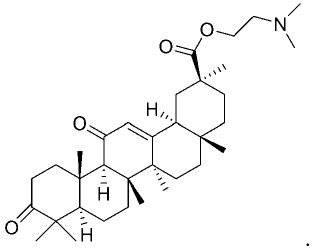

Provided in the present disclosure is a glycyrrhetinic acid derivative having a structure as shown in formula (I), formula (II), or formula (III), or a pharmaceutically acceptable salt thereof: 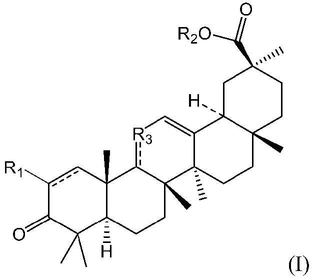
wherein, in the compound of formula (I), R₁ is selected from hydrogen, halogen, hydroxyl, cyano, methoxy, amino, or haloalkyl;
R₂ is selected from -(CH₂)ₙ-NR₂₁R₂₂, where R₂₁ and R₂₂ may be the same or different and are each independently selected from hydrogen and C1-C6 alkyl, or R₂₁ and R₂₂, together with the nitrogen atom to which they are attached, form a 5- to 6-membered heterocyclic group, which heterocyclic group may optionally contain one or more additional heteroatoms selected from O, N, or NH, and n = an integer from 1 to 6;
R₃ is selected from carbonyl or methine;
-̅ -̅ -̅ -̅ is a single bond or a double bond;
----- represents that the H atom attached to the C18-position is in the α-configuration or β-configuration;
   or, 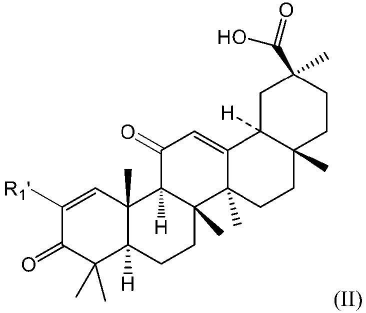
wherein, in the compound of formula (II), R₁' represents Br or Cl;
----- represents that the H atom attached to the C18-position is in the α-configuration or β-configuration;
   or, 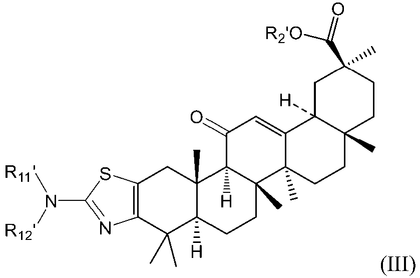
wherein, in the compound of formula (III), R₁₁' and R₁₂' are each independently selected from C1-C6 alkyl;
R₂' is selected from hydrogen or -(CH₂)ₙ-NR₂₁'R₂₂', where R₂₁' and R₂₂' may be the same or different and are each independently selected from hydrogen and C1-C6 alkyl, or R₂₁' and R₂₂', together with the nitrogen atom to which they are attached, form a 5- to 6-membered heterocyclic group, which heterocyclic group may optionally contain one or more additional heteroatoms selected from O, N, or NH, and n = an integer from 1 to 6;
----- represents that the H atom attached to the C18-position is in the α-configuration or β-configuration;
wherein the compound of formula (I) is not selected from 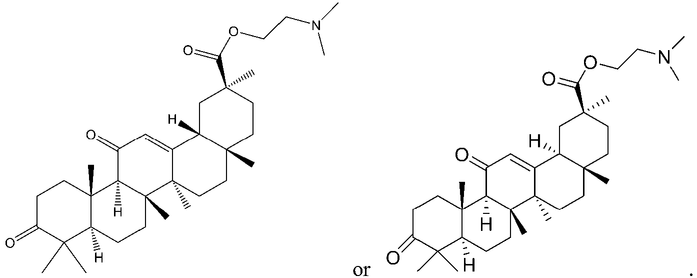
Further, in the compound of formula (I), R₁ is selected from hydrogen or cyano;
R₂ is selected from -(CH₂)ₙ-NR₂₁R₂₂, where R₂₁ and R₂₂ may be the same or different and are each independently selected from C1-C3 alkyl, or R₂₁ and R₂₂, together with the nitrogen atom to which they are attached, form a 5- to 6-membered heterocyclic group, which heterocyclic group may optionally contain one or more additional heteroatoms selected from O, and n = an integer from 2 to 5;
R₃ is selected from carbonyl or methine;
-̅ -̅ -̅ -̅ is a single bond or a double bond;
----- represents that the H atom attached to the C18-position is in the α-configuration or β-configuration;
preferably, in the compound of formula (I), R₁ is selected from hydrogen or cyano;
R₂ is selected from -(CH₂)ₙ-NR₂₁R₂₂, where R₂₁ and R₂₂ may be the same or different and are each independently selected from methyl and ethyl, or R₂₁ and R₂₂, together with the nitrogen atom to which they are attached, form a 5- to 6-membered heterocyclic group, namely 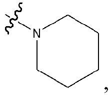 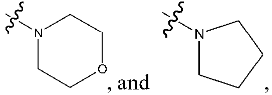 and n = an integer from 2 to 5;
R₃ is selected from carbonyl or methine;
-̅ -̅ -̅ -̅ is a single bond or a double bond;
----- represents that the H atom attached to the C18-position is in the α-configuration or β-configuration.

In some embodiments, in the compound of formula (I), when R₁ is hydrogen, R₂ is -(CH₂)ₙ-NR₂₁R₂₂, where R₂₁ and R₂₂ may be the same or different and are each independently selected from C2-C3 alkyl, or R₂₁ and R₂₂, together with the nitrogen atom to which they are attached, form a 5- to 6-membered heterocyclic group (e.g., a 5- to 6-membered heterocycloalkyl group), n = an integer from 2 to 5, and the heterocyclic group may optionally additionally contain one or more (e.g., 1 to 2) heteroatoms selected from O or N; R₃ is carbonyl; -̅ -̅ -̅ -̅ is a single bond; and ----- represents that the H atom attached to the C18-position is in the α-configuration or β-configuration;
or, when R₁ is hydroxyl, cyano, or amino (preferably cyano), R₂ is -(CH₂)ₙ-NR₂₁R₂₂, where R₂₁ and R₂₂ may be the same or different and are each independently selected from C1-C3 alkyl, and n = an integer from 2 to 5 (preferably n = 2 or 3; preferably, R₂ is -(CH₂)₂-N(C1-C2 alkyl)(C1-C2 alkyl)); R₃ is methine or carbonyl; -̅ -̅ -̅ -̅ is a single bond or a double bond; and ----- represents that the H atom attached to the C18-position is in the α-configuration or β-configuration.

In some embodiments, in the compound of formula (I), when R₁ is hydrogen, R₂ is -(CH₂)ₙ-NR₂₁R₂₂, where R₂₁ and R₂₂ may be the same or different and are each independently selected from C2-C3 alkyl, or R₂₁ and R₂₂, together with the nitrogen atom to which they are attached, form a 5- to 6-membered heterocycloalkyl group, n = an integer from 2 to 5, and the heterocycloalkyl group may optionally additionally contain one or more (e.g., 1 to 2) heteroatoms selected from O or N; R₃ is carbonyl; -̅ -̅ -̅ -̅ is a single bond; and ----- represents that the H atom attached to the C18-position is in the α-configuration or β-configuration;

or, when R₁ is cyano or amino (preferably cyano), R₂ is -(CH₂)ₙ-NR₂₁R₂₂, where R₂₁ and R₂₂ may be the same or different and are each independently selected from C1-C3 alkyl, and n = an integer from 2 to 5 (preferably n = 2 or 3; preferably, R₂ is -(CH₂)₂-N(C1-C2 alkyl)(C1-C2 alkyl)); R₃ is methine or carbonyl; -̅ -̅ -̅ -̅ is a single bond or a double bond (preferably, -̅ -̅ -̅ -̅ attached to R₃ is a single bond, and the other -̅ -̅ -̅ -̅ is a double bond); and ----- represents that the H atom attached to the C18-position is in the α-configuration or β-configuration.

Further, in the compound of formula (I), R₁ is selected from hydrogen or cyano;
R₂ is selected from -(CH₂)ₙ-NR₂₁R₂₂, where R₂₁ and R₂₂ may be the same or different and are each independently selected from methyl and ethyl, or R₂₁ and R₂₂, together with the nitrogen atom to which they are attached, form a 5- to 6-membered heterocyclic group, namely 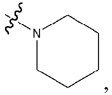 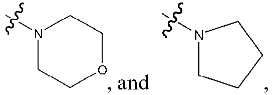 and n = an integer from 2 to 5;
R₃ is selected from carbonyl;
-̅ -̅ -̅ -̅ is a single bond or a double bond;
----- represents that the H atom attached to the C18-position is in the α-configuration or β-configuration;
preferably, in the compound of formula (I), R₁ is selected from hydrogen or cyano;
R₂ is selected from -(CH₂)ₙ-NR₂₁R₂₂, where R₂₁ and R₂₂ may be the same or different and are each independently selected from ethyl, and n = 2;
R₃ is selected from carbonyl;
-̅ -̅ -̅ -̅ is a single bond or a double bond;
----- represents that the H atom attached to the C18-position is in the α-configuration or β-configuration.

Further, in the compound of formula (I), R₁ is selected from cyano;
R₂ is selected from -(CH₂)ₙ-NR₂₁R₂₂, where R₂₁ and R₂₂ may be the same or different and are each independently selected from methyl and ethyl, and n = 2;
R₃ is selected from methine;
-̅ -̅ -̅ -̅ is a double bond;
----- represents that the H atom attached to the C18-position is in the α-configuration or β-configuration.

In some embodiments, in the compound of formula (I), when R₁ is hydrogen, R₂ is -(CH₂)ₙ-NR₂₁R₂₂, where R₂₁ and R₂₂ may be the same or different and are each independently selected from C2-C3 alkyl (e.g., ethyl and propyl), or R₂₁ and R₂₂, together with the nitrogen atom to which they are attached, form 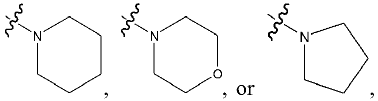 and n = an integer from 2 to 5; R₃ is carbonyl; -̅ -̅ -̅ -̅ is a single bond; and ----- represents that the H atom attached to the C18-position is in the α-configuration or β-configuration;
or, when R₁ is cyano, R₂ is -(CH₂)ₙ-NR₂₁R₂₂, where R₂₁ and R₂₂ may be the same or different and are each independently selected from C1-C3 alkyl (e.g., methyl, ethyl, and propyl), and n = 2 or 3 (preferably, R₂ is -(CH₂)₂-N(C1-C2 alkyl)(C1-C2 alkyl)); R₃ is carbonyl; -̅ -̅ -̅ -̅ is a single bond or a double bond (preferably, -̅ -̅ -̅ -̅ attached to R₃ is a single bond, and the other -̅ -̅ -̅ -̅ is a double bond); and ----- represents that the H atom attached to the C18-position is in the α-configuration or β-configuration.

In some embodiments, in the compound of formula (I), R₁ is hydrogen or cyano; R₂ is -(CH₂)ₙ-NR₂₁R₂₂, where R₂₁ and R₂₂ may be the same or different and are each independently selected from C2-C3 alkyl (e.g., ethyl and propyl), and n = 2 or 3 (preferably, R₂ is -(CH₂)₂-N(C1-C2 alkyl)(C1-C2 alkyl), such as -(CH₂)₂-N(CH₂CH₃)₂); R₃ is carbonyl; -̅ -̅ -̅ -̅ is a single bond or a double bond (preferably, -̅ -̅ -̅ -̅ attached to R₃ is a single bond, and the other -̅ -̅ -̅ -̅ is a single bond or a double bond); and ----- represents that the H atom attached to the C18-position is in the α-configuration or β-configuration.

In some embodiments, in the compound of formula (III), R₁₁' and R₁₂' are each independently selected from C1-C5 alkyl, C1-C4 alkyl, or C1-C3 alkyl (e.g., methyl, ethyl, and propyl);
R₂' is selected from hydrogen or -(CH₂)ₙ-NR₂₁'R₂₂', where R₂₁' and R₂₂' may be the same or different and are each independently selected from C1-C5 alkyl (preferably C1-C4 alkyl or C1-C3 alkyl, such as methyl, ethyl, and propyl), and n = an integer from 1 to 3 (preferably n = 2 or 3; preferably, R₂' is -(CH₂)₂-N(C1-C2 alkyl)(C1-C2 alkyl), such as -(CH₂)₂-N(CH₂CH₃)₂);
----- represents that the H atom attached to the C18-position is in the α-configuration or β-configuration.

Further, in the compound of formula (III), R₁₁" and R₁₂' are each independently selected from C1-C3 alkyl;
R₂' is selected from hydrogen or -(CH₂)ₙ-NR₂₁'R₂₂', where R₂₁' and R₂₂' may be the same or different and are each independently selected from C1-C3 alkyl, and n = an integer from 1 to 3;
----- represents that the H atom attached to the C18-position is in the α-configuration or β-configuration;
preferably, in the compound of formula (III), R₁₁' and R₁₂' are each independently selected from methyl, ethyl, or propyl;
R₂' is selected from hydrogen or -(CH₂)ₙ-NR₂₁'R₂₂', where R₂₁' and R₂₂' may be the same or different and are each independently selected from methyl, ethyl, or propyl, preferably ethyl, and n = 2;
----- represents that the H atom attached to the C18-position is in the α-configuration or β-configuration;
further preferably, in the compound of formula (III), R₁₁' and R₁₂' are each independently selected from methyl, ethyl, or propyl;
R₂' is selected from hydrogen;
----- represents that the H atom attached to the C18-position is in the α-configuration or β-configuration.

Further, the compound of formula (I) is selected from the following compounds: 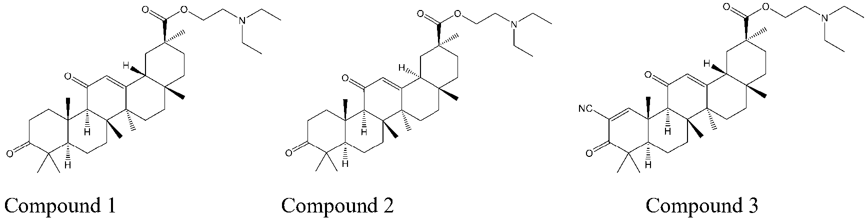 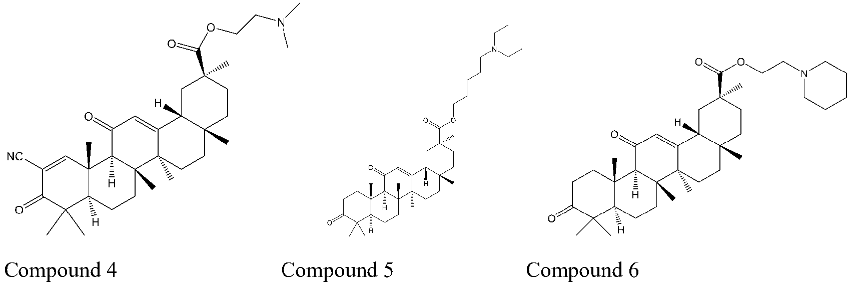 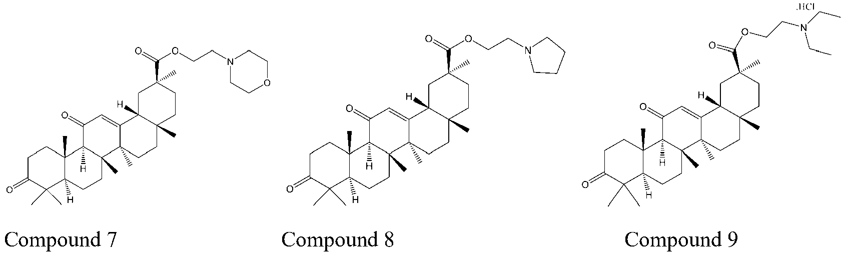 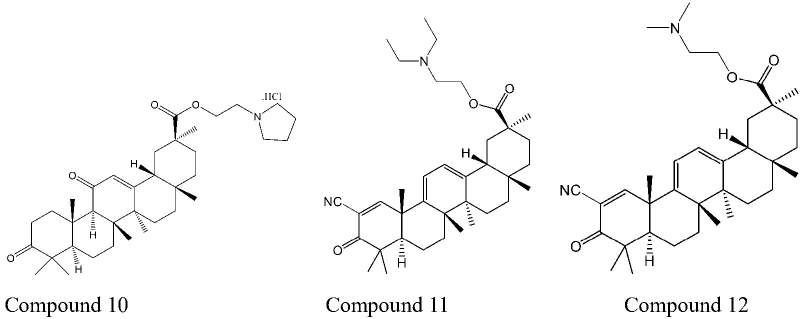 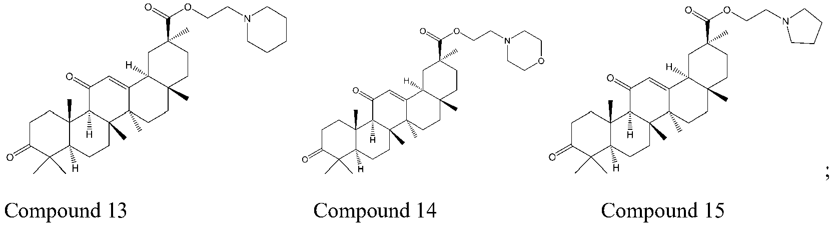 preferably Compound 1, Compound 2, Compound 3, Compound 4, Compound 5, Compound 6, Compound 7, Compound 8, Compound 9, Compound 10, Compound 13, Compound 14, and Compound 15;
further preferably Compound 1, Compound 2, Compound 3, and Compound 9.

Further, the compound of formula (II) is selected from the following compounds: 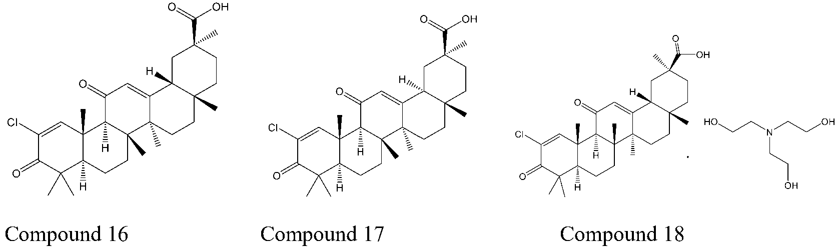 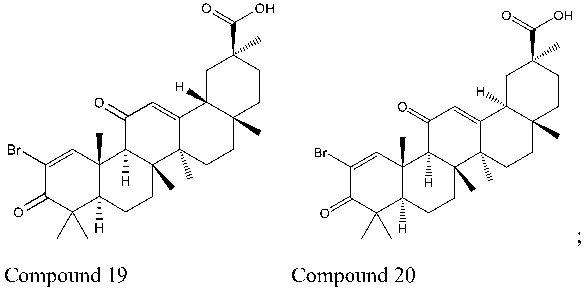 preferably Compound 16, Compound 17, and Compound 18.

Further, the compound of formula (III) is selected from the following compounds: 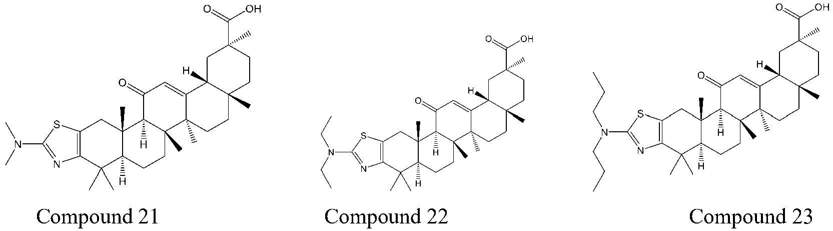 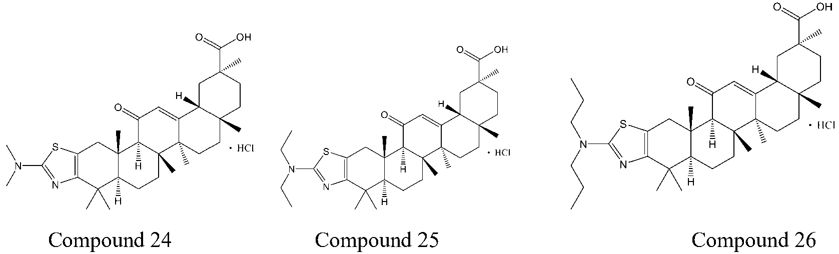 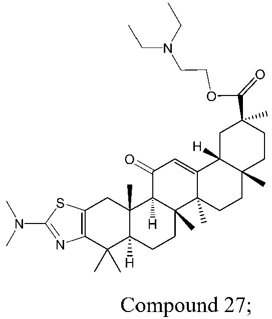 preferably Compound 21, Compound 22, and Compound 23.

Also provided in the present disclosure is a pharmaceutical composition, comprising the glycyrrhetinic acid derivative as described above (i.e., a compound of formula (I), formula (II), or formula (III), or a pharmaceutically acceptable salt thereof), and optionally one or more pharmaceutically acceptable carriers, diluents, excipients, or adjuvants.

Further, the pharmaceutical composition is a solid oral formulation, a liquid oral formulation, an injection, or a topical formulation.

Further, the solid oral formulation is selected from a tablet, capsule, dry suspension, or granule; the liquid oral formulation is selected from a syrup, oral solution, oral suspension, or oral emulsion; the injection is selected from an injection solution, sterile powder for injection, or concentrated solution for injection; the topical formulation is selected from an ointment, cream, gel, latex, paste, liniment, paint, tincture, lotion, film-forming agent, spray, suppository, foam, or enema.

Further provided in the present disclosure is the use of the glycyrrhetinic acid derivative as described above (i.e., a compound of formula (I), formula (II), or formula (III), or a pharmaceutically acceptable salt thereof) or the pharmaceutical composition as described above in the manufacture of a medicament for the treatment of inflammatory diseases or autoimmune diseases. Alternatively, further provided in the present disclosure is the glycyrrhetinic acid derivative as described above (i.e., a compound of formula (I), formula (II), or formula (III), or a pharmaceutically acceptable salt thereof) or the pharmaceutical composition as described above for use as a medicament in the treatment of inflammatory diseases or autoimmune diseases. Alternatively, further provided in the present disclosure is a method for treating inflammatory diseases or autoimmune diseases, comprising administering to a subject in need thereof a therapeutically effective amount of the glycyrrhetinic acid derivative as described above (i.e., a compound of formula (I), formula (II), or formula (III), or a pharmaceutically acceptable salt thereof) or the pharmaceutical composition as described above.

Further, the diseases are selected from dermatitis, eczema, atopic dermatitis, seborrhoeic dermatitis, hormone-dependent dermatitis, photosensitive dermatitis, chloasma, psoriasis, urticaria, prurigo nodularis, pityriasis rosea, inflammatory bowel disease, lupus erythematosus, alopecia areata, pemphigus, bullous pemphigoid, chronic nephritis, asthma, rhinitis, skin lesions caused by radiotherapy (including, but not limited to, radiation dermatitis), burns, scalds, or solar dermatitis. As used herein, "therapeutically effective amount" or "effective amount" refers to an amount sufficient to produce a desired response (including preventing or slowing the onset of a disease, reducing the severity of a disease, ameliorating a patient's condition, inducing an immune response, delaying or at least slowing the progression of a disease, or curing) in a patient or individual in need thereof, and can be determined by a person skilled in the art based on the patient's or individual's age, gender, weight, physical condition, family history, disease risk, etc., in combination with the present disclosure.

Compared with the prior art, the present disclosure has the following beneficial effects:
The compounds of the present disclosure exhibit significant inhibitory activity in the RAW264.7 cell inflammation model, ear swelling model, atopic dermatitis model, radiation dermatitis model, pruritus model, etc., with remarkable anti-inflammatory effects. They exhibit prominent drug distribution in the colon, can significantly reduce intracellular ROS levels in a ROS release assay, and thus can be used in the treatment of various inflammatory diseases or autoimmune diseases. Moreover, the compounds of the present disclosure, as topical formulations, exhibit significant advantages such as low skin irritation and high safety.

### Specific Implementations

### Definitions

Unless otherwise stated, the following terms used in the specification and claims have the meanings set forth below. A specific term, without being specially defined, shall not be regarded as ambiguous or unclear, but shall be construed in accordance with the conventional meaning in the art.

"Alkyl" refers to a saturated hydrocarbon group, which may be linear or branched. As used herein, "C1-C6 alkyl" refers to a linear or branched alkyl group consisting of 1 to 6 (e.g., 1, 2, 3, 4, 5, 6, or a range formed by any two of the foregoing values) carbon atoms. Typical alkyl groups include, but are not limited to, methyl, ethyl, n-propyl, isopropyl, n-butyl, isobutyl, tert-butyl, n-pentyl, isopentyl, neopentyl, n-hexyl, etc.

"Heteroatom" refers to any atom other than carbon and hydrogen that can be covalently bonded to a carbon atom. Common heteroatoms include, but are not limited to, O, S, and N, etc.

"Heterocyclic group" refers to a monocyclic, bridged, or spirocyclic monovalent radical that is fully saturated or partially unsaturated (but not fully unsaturated, e.g., having 1 or 2 double bonds), wherein at least one (e.g., 1, 2, 3, or 4) ring atom is a heteroatom selected from N, O, and S, and the remaining ring atoms are C. For example, a 5- to 6-membered heterocyclic group includes a heterocycloalkyl or heterocycloalkenyl group containing 1 to 2 heteroatoms selected from N, O, and S. "Heterocycloalkyl" refers to a saturated heterocyclic group.

"Aromatic heterocyclic group", also referred to as "heteroaryl", refers to an aromatic cyclic structure formed by replacing one or more carbon atoms in an aromatic ring with heteroatoms. For example, a 5- to 6-membered aromatic heterocyclic group refers to an aromatic cyclic group containing heteroatoms and consisting of 5 or 6 ring atoms.

"Halogen" refers to fluorine, chlorine, bromine, or iodine.

"Oxo" refers to =O.

"Haloalkyl" refers to an alkyl group in which at least one hydrogen atom is replaced by a halogen atom. For example, "C1-C6 haloalkyl" refers to a linear or branched alkyl group consisting of 1 to 6 carbon atoms, wherein at least one hydrogen atom on the alkyl group is replaced by any halogen atom.

"Optional" or "optionally" means that the subsequently described event or circumstance may, but does not necessarily, occur, and the description includes instances where the event or circumstance occurs and instances where it does not. For example, "optionally substituted" includes substituted or unsubstituted, and "optionally comprises" encompasses comprising and not comprising.

"Pharmaceutically acceptable" refers to those compounds, materials, compositions, and/or dosage forms that, within the scope of sound medical judgment, are suitable for use in contact with human and animal tissues without undue toxicity, irritation, allergic responses, or other problems or complications, commensurate with a reasonable benefit/risk ratio.

Unless otherwise indicated, the terms "comprise", "comprises", and "comprising", or their equivalents (contain, contains, containing, include, includes, including), as used herein, are openended expressions and mean that, in addition to the recited elements, components, and steps, other unrecited elements, components, and steps may also be encompassed.

Unless otherwise indicated, all numbers as used herein to express quantities of ingredients, measured values, or reaction conditions are to be understood as being modified in all instances by the term "about". When linked to a percentage, the term "about" may mean, for example, +1%, preferably +0.5%, more preferably 0.1%.

Unless the context clearly dictates otherwise, the singular terms used herein encompass plural referents and vice versa. Similarly, unless the context clearly dictates otherwise, the word "or" as used herein is intended to include "and".

As used herein, and unless otherwise indicated, the term "treatment" refers to statistically significant curing, alleviating, relieving, slowing, mitigating, or ameliorating a disease or disease-related symptoms, or preventing, delaying, inhibiting, arresting, or stopping the onset or further progression of a disease or related symptoms.

The term "subject" encompasses any vertebrate, e.g., mammals and non-mammals, such as humans, non-human primates (e.g., chimpanzees, rhesus monkeys, cynomolgus monkeys, etc.), sheep, dogs, cats, horses, cattle, chickens, pigs, mice, etc., preferably humans.

As used herein, and unless otherwise indicated, "C18-position" represents the carbon atom at the C18 position in the corresponding glycyrrhetinic acid structure in the compounds of formula (I), formula (II), or formula (III) of the present disclosure.

### Examples

Embodiments of the present disclosure will be described in detail below with reference to the examples. However, those skilled in the art will understand that the following examples are for illustrative purposes only and shall not be construed as limiting the scope of the present disclosure. Examples, where specific conditions are not specified, are implemented in accordance with general conditions. All of the used reagents or instruments, whose manufacturers are not specified, are conventional commercially-available products.

Herein, DMAP: 4-dimethylaminopyridine, EDCI: 1-ethyl-3-(3-dimethylaminopropyl)carbodiimide, IBX: 2-iodoxybenzoic acid, DMSO: dimethyl sulfoxide, DMF: dimethylformamide.

### Preparation of specific compounds having a structure as shown in formula (I) or a pharmaceutically acceptable salt thereof:

### Example 1

### 2-(Diethylamino)ethyl (2S,4aS,6aS,6bR,8aR,12aS,12bR,14bR)-2,4a,6a,6b,9,9,12a-heptamethyl-10,13-dioxo-1,2,3,4,4a,5,6,6a,6b,7,8,8a,9,10,11, 12,12a,12b,13,14b-icosahydropicene-2-carboxylate

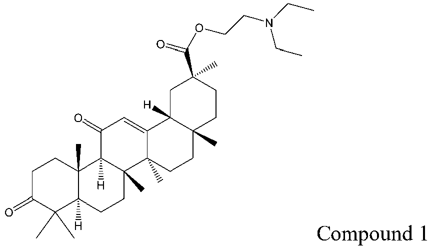

### Synthesis Procedure:

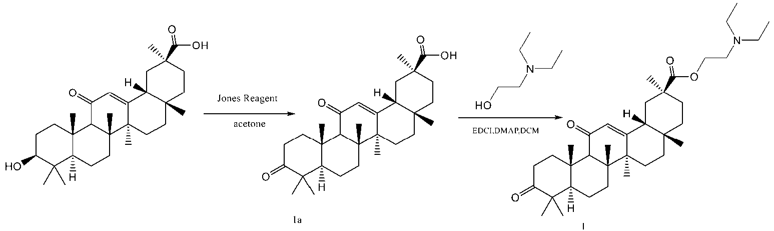

Preparation of Intermediate 1a: 18β-Glycyrrhetinic acid (10 g, 21 mmol) was dissolved in acetone (120 mL), and 12 mL of Jones reagent was slowly added dropwise under ice-bath conditions. The reaction was carried out under ice-bath conditions and monitored by TLC until no 18β-glycyrrhetinic acid remained. The reaction mixture was then poured into 300 mL of water, and a large amount of white precipitate formed. The mixture was filtered, and the filter cake was washed several times with copious water until the pH of the washings reached about 6 to 7, followed by drying under reduced pressure to afford Intermediate 1a (9 g, yield 90%). MS m/z (ESI): 469.42 [M+H]⁺.

Preparation of Compound 1: Intermediate 1a (5 g, 11 mmol), diethylaminoethanol (1.5 g, 13 mmol), DMAP (0.26 g, 2.1 mmol), and EDCI (2.5 g, 13 mmol) were dissolved in dichloromethane (85 mL). The reaction was carried out at room temperature and monitored by TLC until no Intermediate 1a remained. Dichloromethane (100 mL) was then added to the reaction mixture. The mixture was washed successively with 10% citric acid solution (100 mL), saturated sodium bicarbonate solution (100 mL), and water (100 mL), dried over anhydrous sodium sulfate, and concentrated in vacuo.

The concentrate was purified by silica gel column chromatography (petroleum ether: tetrahydrofuran = 10:1) to afford Compound 1 (4 g, yield 66%). MS m/z (ESI): 568.47 [M+H]⁺. ¹H-NMR(400 MHz, DMSO-d₆): δ 5.52 (s,1H), 4.13 (m,2H), 2.70 (m,1H), 2.60(m,2H), 2.54 (m,2H), 2,46 (m,2H), 2.30 (m,1H), 2.09 (m,2H), 1.73 (m,5H), 1.47 (m,4H), 1.36 (m,6H), 1.29 (m,2H), 1.13 (m, 4H), 1.09 (m,6H), 0.96 (m,14H), 0.75 (s,3H).

### Example 2

### 2-(Diethylamino)ethyl (2S,4aS,6aS,6bR,8aR,12aS,12bR,14bS)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate

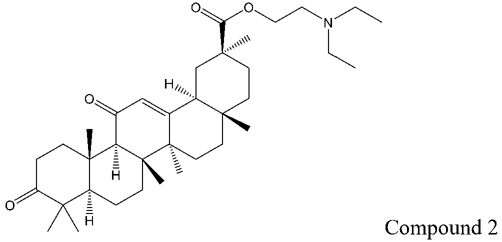

### Synthesis Procedure:

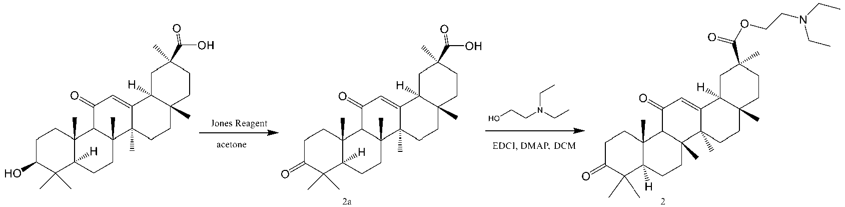

Preparation of Intermediate 2a: The preparation procedure was performed with reference to Intermediate 1a in Example 1, except that 18-β glycyrrhetinic acid was replaced with 18-α glycyrrhetinic acid, and the other steps were identical. MS m/z (ESI): 469.42 [M+H]⁺.

Preparation of Compound 2: The preparation procedure was performed with reference to Compound 1 in Example 1, except that Intermediate 1a was replaced with Intermediate 2a, and the other steps were identical. MS m/z (ESI): 568.47 [M+H]⁺. ¹H-NMR (400 MHz, DMSO-d6): δ 5.52 (s, 1H), 4.13 (m, 2H), 2.70 (m, 1H), 2.58(m, 2H), 2.51 (m, 2H), 2,43 (m, 2H), 2.28 (m, 1H), 2.04 (m, 3H), 1.73 (m, 4H), 1.47 (m, 4H), 1.36 (m, 6H), 1.29 (m, 2H), 1.13 (m, 4H), 1.09 (m, 6H), 0.96 (m, 14H), 0.75 (s, 3H).

### Example 3

### 2-(Diethylamino)ethyl (2S,4aS,6aS,6bR,8aR,12aS,12bR,14bR)-11-cyano-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylate

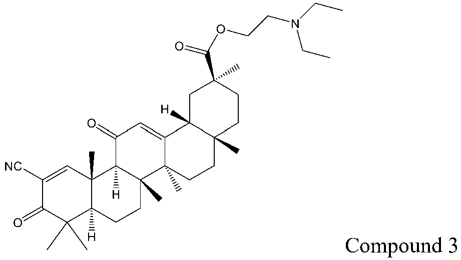

### Synthesis Procedure:

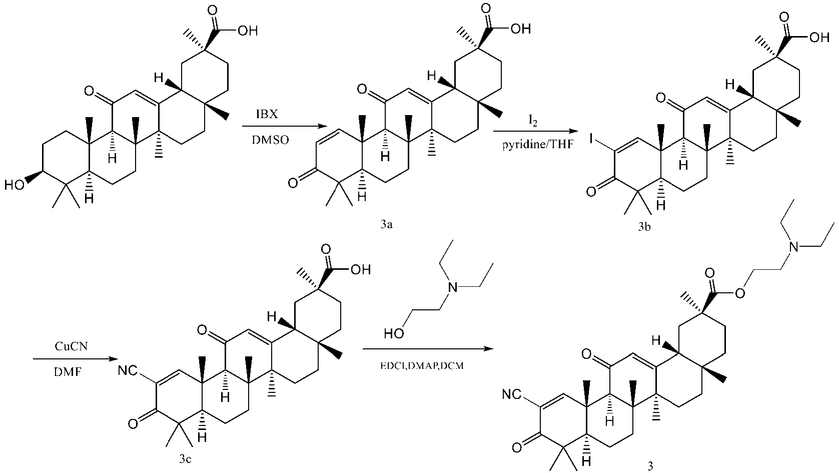

Preparation of Intermediate 3a: 18β-Glycyrrhetinic acid (10 g, 21 mmol) and IBX (17.6 g, 63 mmol) were dissolved in DMSO (100 mL). The reaction was carried out at 85°C and monitored by TLC until no 18β-glycyrrhetinic acid remained. After cooling to room temperature, the reaction mixture was poured into 800 mL of water, and a large amount of white precipitate formed. The mixture was filtered, and the filter cake was washed three times with water, followed by drying under reduced pressure to afford Intermediate 3a (9 g, yield 91%). MS m/z (ESI): 467.42 [M+H]⁺.

Preparation of Intermediate 3b: Intermediate 3a (4 g, 8.6 mmol) was dissolved in tetrahydrofuran (100 mL), followed by the addition of I₂ (13 g, 51 mmol) and pyridine (6.1 g, 77 mmol). The reaction was carried out under reflux and monitored by TLC until no Intermediate 3a remained. After cooling to room temperature, most of the tetrahydrofuran was evaporated under reduced pressure. The mixture was washed with sodium thiosulfate solution until it turned golden yellow, extracted with ethyl acetate, dried over anhydrous sodium sulfate, and concentrated in vacuo. The concentrate was purified by silica gel column chromatography (tetrahydrofuran) to afford Intermediate 3b (4.5 g, yield 88%). MS m/z (ESI): 593.56 [M+H]⁺.

Preparation of Intermediate 3c: Intermediate 3b (4 g, 6.8 mmol) was dissolved in DMF (50 mL), followed by the addition of cuprous cyanide (1.2 g, 13 mmol). The reaction was carried out at 140°C and monitored by TLC until no Intermediate 3b remained. After cooling to room temperature, water (100 mL) was added to the reaction mixture, and a solid precipitated. The mixture was filtered with suction, and the filtrate was extracted with ethyl acetate, dried over anhydrous sodium sulfate, and concentrated in vacuo. The concentrate was purified by silica gel column chromatography (tetrahydrofuran) to afford Intermediate 3c (2.5 g, yield 75%). MS m/z (ESI): 492.31 [M+H]⁺.

Preparation of Compound 3: Intermediate 3c (2 g, 4.1 mmol), diethylaminoethanol (0.57 g, 4.9 mmol), DMAP (0.1 g, 0.8 mmol), and EDCI (0.93 g, 4.9 mmol) were dissolved in dichloromethane (30 mL). The reaction was carried out at room temperature and monitored by TLC until no Intermediate 3c remained. Dichloromethane (40 mL) was then added to the reaction mixture. The mixture was washed successively with 10% citric acid solution (40 mL), saturated sodium bicarbonate solution (40 mL), and water (40 mL), dried over anhydrous sodium sulfate, and concentrated in vacuo. The concentrate was purified by silica gel column chromatography (petroleum ether: tetrahydrofuran = 10:1) to afford Compound 3 (1.5 g, yield 62%). MS m/z (ESI): 591.45 [M+H]⁺. ¹H-NMR (400 MHz, DMSO-d6): δ 8.47 (s, 1H), 5.63 (s, 1H), 4.17 (m, 2H), 2.96 (m, 1H), 2.62(m, 2H), 2.48 (m, 2H), 2.13 (m, 2H), 1.77 (m, 7H), 1.57 (m, 2H), 1.37 (m, 9H), 1.29 (m, 2H), 1.17 (m, 13H), 0.99 (m, 7H), 0.76 (s, 3H).

### Example 4

### 2-(Dimethylamino)ethyl (2S,4aS,6aS,6bR,8aR,12aS,12bR,14bR)-11-cyano-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylate

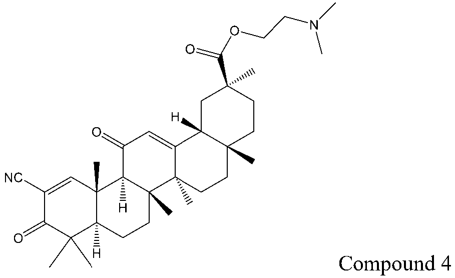

### Synthesis Procedure:

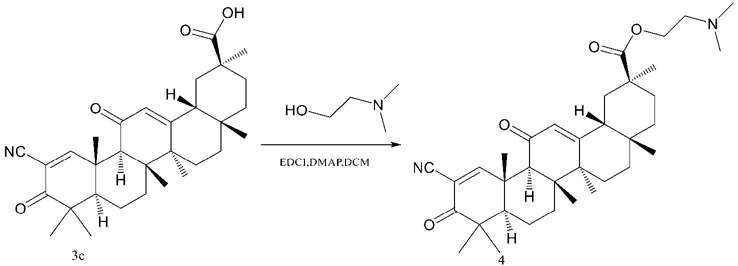

Preparation of Compound 4: The preparation procedure was performed with reference to Compound 3 in Example 3, except that diethylaminoethanol was replaced with dimethylaminoethanol, and the other steps were identical. The concentrate was purified by silica gel column chromatography (petroleum ether: tetrahydrofuran = 10:1) to afford Compound 4 (1.3 g, yield 57%). MS m/z (ESI): 563.36[M+H]⁺. ¹H-NMR (400 MHz, DMSO-d6):δ 8.43 (s, 1H), 5.60 (s, 1H), 3.95 (m, 2H), 2.94 (m, 1H), 2.03 (m, 2H), 1.74 (m, 7H), 1.56 (m, 2H), 1.36 (m, 9H), 1.27 (m, 2H), 1.14 (m, 13H), 0.97 (m, 7H), 0.86 (s, 3H).

### Example 5

### 5-(Diethylamino)pentyl (2S,4aS,6aS,6bR,8aR,12aS,12bR,14bR)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate

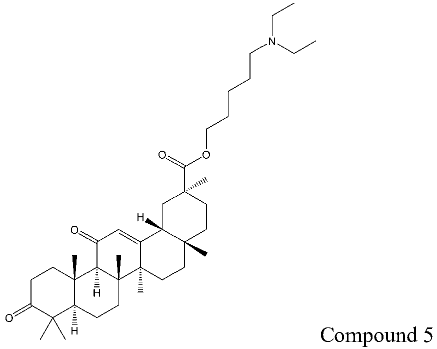

### Synthesis Procedure:

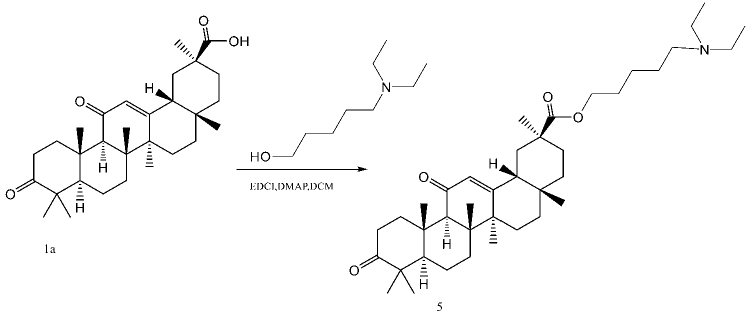

Preparation of Compound 5: The preparation procedure was performed with reference to Compound 1 in Example 1, except that diethylaminoethanol was replaced with diethylaminopentanol, and the other steps were identical. The concentrate was purified by silica gel column chromatography (petroleum ether: tetrahydrofuran = 10:1) to afford Compound 5 (2.1 g, yield 32%). MS m/z (ESI): 610.42[M+H]⁺. ¹H-NMR (400 MHz, DMSO-d6): δ 5.62 (s, 1H), 4.63 (m, 2H), 2.75 (m, 1H), 2,41 (m, 2H), 2.28 (m, 1H), 2.19 (m, 2H), 1.64 (m, 5H), 1.40 (m, 4H), 1.32 (m, 6H), 1.23 (m, 2H), 1.13 (m, 8H), 1.03 (m, 12H), 0.86 (m, 14H), 0.70 (s, 3H).

### Example 6

### 2-(Piperidin-1-yl)ethyl (2S,4aS,6aS,6bR,8aR,12aS,12bR,14bR)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate

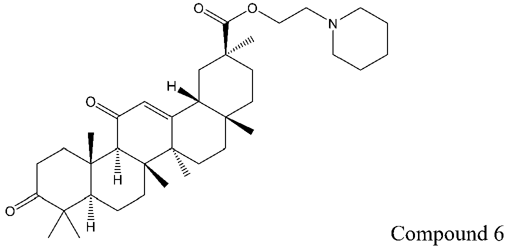

### Synthesis Procedure:

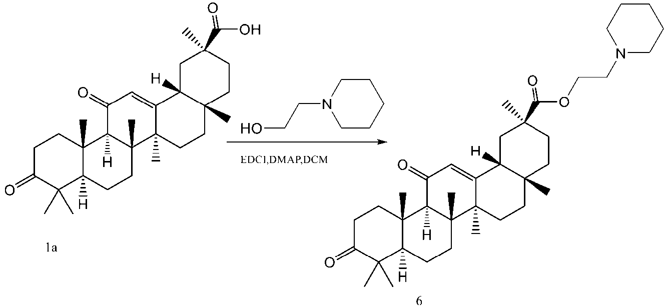

Preparation of Compound 6: The preparation procedure was performed with reference to Compound 1 in Example 1, except that diethylaminoethanol was replaced with 2-piperidinoethanol, and the other steps were identical. The concentrate was purified by silica gel column chromatography (petroleum ether: tetrahydrofuran = 10:1) to afford Compound 6 (2.0 g, yield 32%). MS m/z (ESI): 580.41[M+H]+. ¹H-NMR (400 MHz, DMSO-d6): δ 5.62 (s, 1H), 4.07 (m, 2H), 2.82 (m, 1H), 2.68(m, 2H), 2.49 (m, 4H), 2.36 (m, 1H), 2.18 (m, 2H), 1.97 (m, 5H), 1.45 (m, 8H), 1.38 (m, 8H), 1.31 (m, 2H), 1.16 (m, 4H), 1.03 (m, 14H), 0.75 (s, 3H).

### Example 7

### 2-Morpholinoethyl (2S,4aS,6aS,6bR,8aR,12aS,12bR,14bR)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate

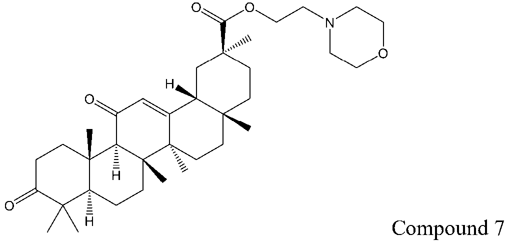

### Synthesis Procedure:

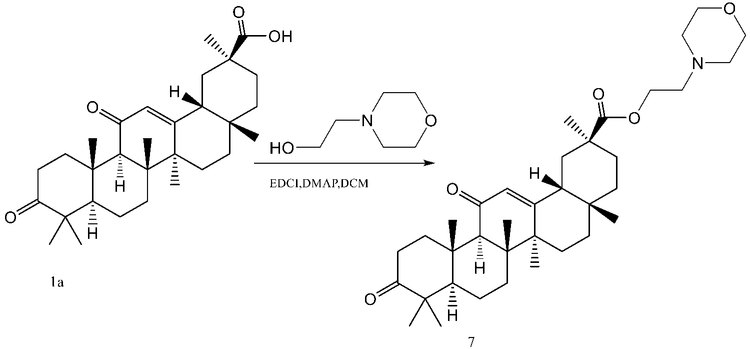

Preparation of Compound 7: The preparation procedure was performed with reference to Compound 1 in Example 1, except that diethylaminoethanol was replaced with 2-morpholinoethanol, and the other steps were identical. The concentrate was purified by silica gel column chromatography (petroleum ether: tetrahydrofuran = 10:1) to afford Compound 7 (2.4 g, yield 38%). MS m/z (ESI): 582.43[M+H]⁺. ¹H-NMR (400 MHz, DMSO-d6): δ 5.98 (s, 1H), 4.13 (m, 2H), 3.57 (m, 4H), 3.03 (m, 2H), 2.82 (m, 1H), 2.50 (m, 4H), 2.41 (m, 1H), 2.27 (m, 2H), 1.99 (m, 5H), 1.64 (m, 4H), 1.41 (m, 6H), 1.35 (m, 2H), 1.23 (m, 4H), 1.05 (m, 14H), 0.89 (s, 3H).

### Example 8

### 2-(Pyrrolidin-1-yl)ethyl (2S,4aS,6aS,6bR,8aR,12aS,12bR,14bR)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate

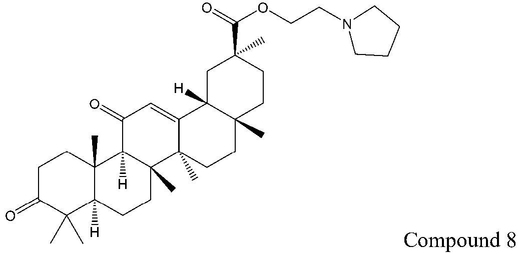

### Synthesis Procedure:

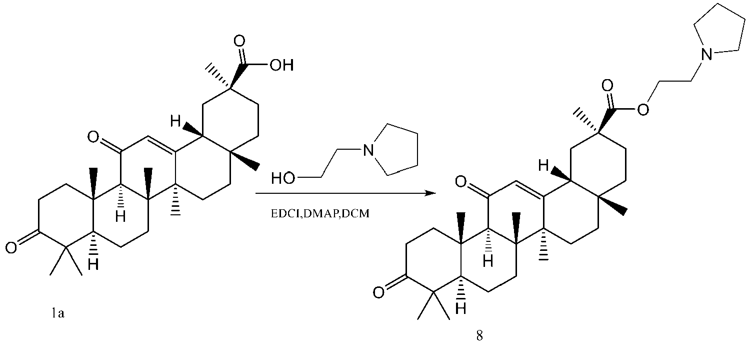

Preparation of Compound 8: The preparation procedure was performed with reference to Compound 1 in Example 1, except that diethylaminoethanol was replaced with 2-pyrrolidinoethanol, and the other steps were identical. The concentrate was purified by silica gel column chromatography (petroleum ether: tetrahydrofuran = 10:1) to afford Compound 8 (2.2 g, yield 36%). MS m/z (ESI): 566.45[M+H]⁺. ¹H-NMR (400 MHz, DMSO-d6):δ 5.92 (s, 1H), 4.06 (m, 2H), 3.01 (m, 2H), 2.72 (m, 1H), 2.51 (m, 4H), 2.41 (m, 1H), 2.21 (m, 2H), 1.92 (m, 5H), 1.64 (m, 8H), 1.43 (m, 6H), 1.39 (m, 2H), 1.22 (m, 4H), 1.09 (m, 14H), 0.88 (s, 3H).

### Example 9

### 2-(Diethylamino)ethyl (2S,4aS,6aS,6bR,8aR,12aS,12bR,14bR)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate hydrochloride

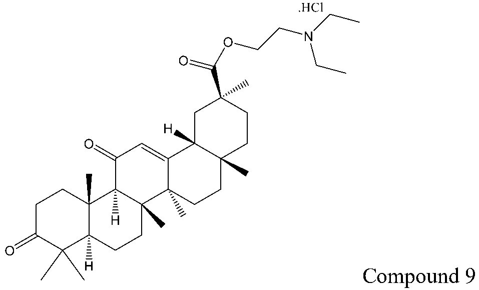

### Synthesis Procedure:

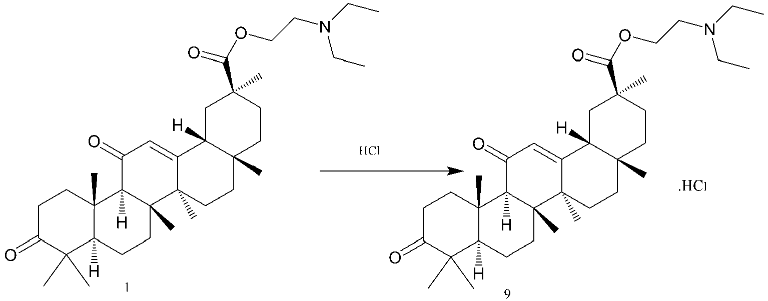

Preparation of Compound 9: Compound 1 (2 g, 3.5 mmol) was dissolved in 200 mL of dichloromethane, and a solution of hydrochloric acid in isopropanol was added dropwise. The reaction was carried out at room temperature for 24 h. Concentration afforded Compound 9 (1.9 g, yield 89%). MS m/z (ESI): 568.43 [M+H]⁺.

### Example 10

### 2-(Pyrrolidin-1-yl)ethyl (2S,4aS,6aS,6bR,8aR,12aS,12bR,14bR)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate hydrochloride

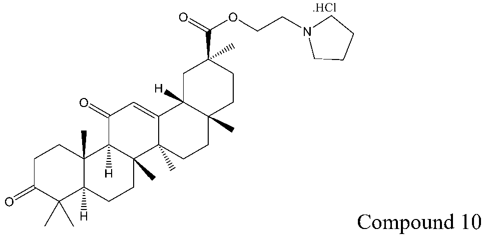

### Synthesis Procedure:

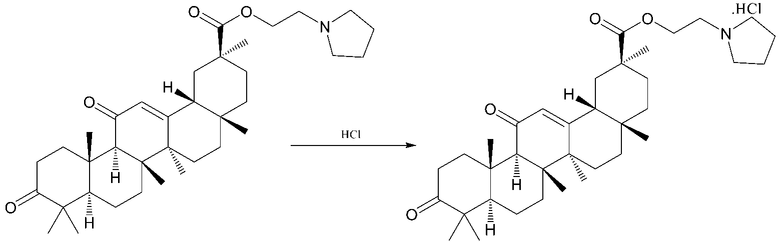

Preparation of Compound 10: Compound 8 (1.5 g, 2.6 mmol) was dissolved in 20 mL of dichloromethane, and a solution of hydrochloric acid in isopropanol was added dropwise. The reaction was carried out at room temperature for 24 h. Concentration afforded Compound 10 (1.3 g, yield 81%). MS m/z (ESI): 566.45[M+H]⁺.

### Example 11

### 2-(Diethylamino)ethyl (2S,4aS,6aR,6bS,8aR,12aS,14bR)-11-cyano-2,4a,6a,6b,9,9,12a-heptamethyl-10-oxo-1,2,3,4,4a,5,6,6a,6b,7,8,8a,9,10,12a,14b-hexadecahydropicene-2-carboxylate

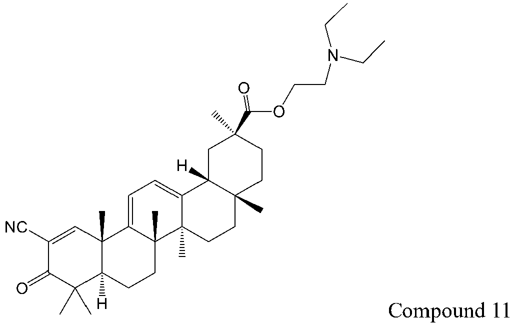

### Synthesis Procedure:

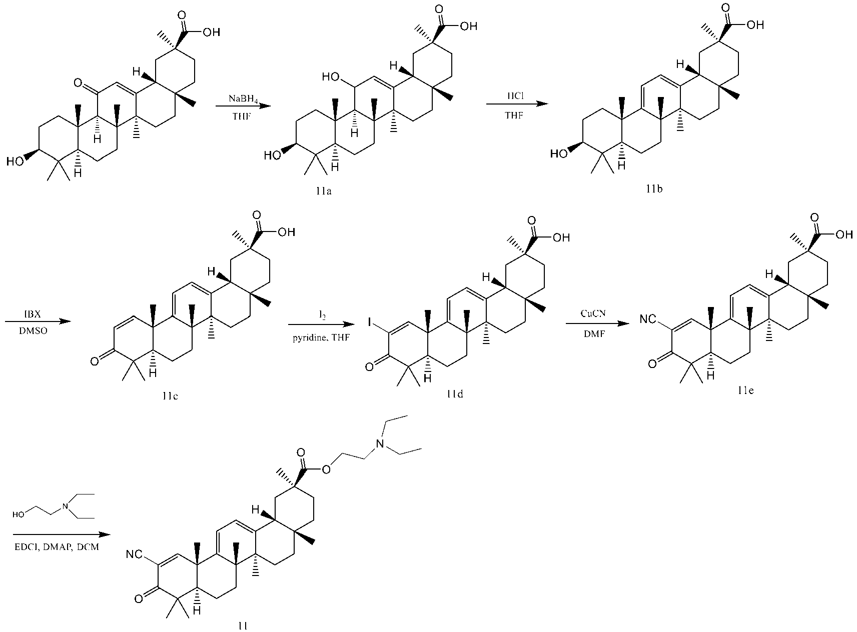

Preparation of Intermediate 11a: 18β-Glycyrrhetinic acid (20 g, 42 mmol) was dissolved in tetrahydrofuran (200 mL), and 64 mL of a solution of sodium borohydride (16.1 g, 425 mmol) in sodium hydroxide (8.4 g, 210 mmol) was slowly added dropwise. After the addition was complete, the reaction was carried out under reflux and monitored by TLC until no 18β-glycyrrhetinic acid remained. After cooling to room temperature, the reaction mixture was poured into 10% sodium dihydrogen phosphate solution (800 mL), extracted with ethyl acetate, dried over anhydrous sodium sulfate, and concentrated in vacuo to afford Intermediate 11a (19 g, yield 96%). MS m/z (ESI): 473.46 [M+H]⁺.

Preparation of Intermediate 11b: Intermediate 11a (18 g, 38 mmol) was dissolved in tetrahydrofuran (200 mL), and a catalytic amount of concentrated hydrochloric acid was slowly added dropwise.

After the addition was complete, the reaction was carried out under reflux and monitored by TLC until no Intermediate 11a remained. The mixture was cooled to room temperature, extracted with ethyl acetate, dried over anhydrous sodium sulfate, and concentrated in vacuo to afford Intermediate 11b (13.8 g, yield 80%). MS m/z (ESI): 455.32 [M+H]⁺.

Preparation of Intermediate 11c: Intermediate 11b (13 g, 28 mmol) and IBX (32 g, 114 mmol) were dissolved in DMSO (450 mL). The reaction was carried out at 85°C and monitored by TLC until no Intermediate 11b remained. After cooling to room temperature, the reaction mixture was poured into 2000 mL of water, and a large amount of white precipitate formed. The mixture was filtered, and the filter cake was washed several times with copious water, followed by drying under reduced pressure to afford Intermediate 11c (11.6 g, yield 90%). MS m/z (ESI): 451.29 [M+H]⁺.

Preparation of Intermediate 11d: Intermediate 11c (11 g, 24.4 mmol) was dissolved in tetrahydrofuran (800 mL), followed by the addition of I₂ (35.7 g, 140 mmol) and pyridine (16.8 g, 212 mmol). The reaction was carried out under reflux and monitored by TLC until no Intermediate 11c remained. After cooling to room temperature, most of the tetrahydrofuran was evaporated under reduced pressure. The mixture was washed with sodium thiosulfate solution until it turned golden yellow, extracted with ethyl acetate, dried over anhydrous sodium sulfate, and concentrated in vacuo. The concentrate was purified by silica gel column chromatography (tetrahydrofuran) to afford Intermediate 11d (12.5 g, yield 89%). MS m/z (ESI): 577.26 [M+H]⁺.

Preparation of Intermediate 11e: Intermediate 11d (12 g, 20.4 mmol) was dissolved in DMF (200 mL), followed by the addition of cuprous cyanide (3.5 g, 39 mmol). The reaction was carried out at 140°C and monitored by TLC until no Intermediate 11d remained. After cooling to room temperature, water (600 mL) was added to the reaction mixture, and a solid precipitated. The mixture was filtered with suction, and the filtrate was extracted with ethyl acetate, dried over anhydrous sodium sulfate, and concentrated in vacuo. The concentrate was purified by silica gel column chromatography (tetrahydrofuran) to afford Intermediate 11e (8.0 g, yield 81%). MS m/z (ESI): 476.32 [M+H]⁺.

Preparation of Compound 11: Intermediate 11e (4 g, 8.4 mmol), diethylaminoethanol (1.2 g, 10.1 mmol), DMAP (0.21 g, 1.7 mmol), and EDCI (1.93 g, 10.1 mmol) were dissolved in dichloromethane (50 mL). The reaction was carried out at room temperature and monitored by TLC until no Intermediate 11e remained. Dichloromethane (30 mL) was then added to the reaction mixture. The mixture was washed successively with 10% citric acid solution (80 mL), saturated sodium bicarbonate solution (80 mL), and water (80 mL), dried over anhydrous sodium sulfate, and concentrated in vacuo. The concentrate was purified by silica gel column chromatography (petroleum ether: tetrahydrofuran = 10:1) to afford Compound 11 (2.1 g, yield 43%). MS m/z (ESI): 575.43 [M+H]⁺. ¹H-NMR (400 MHz, DMSO-d₆): δ 7.82 (s, 1H), 5.66 (d, 1H, J=5.67Hz), 5.52 (d, 1H, J=5.52Hz), 4.87 (m, 2H), 2.96 (m, 4H), 2.08 (m, 3H), 1.96 (m, 1H), 1.78 (m, 5H), 1.55 (m, 4H), 1.29 (m, 4H), 1.18 (s, 3H), 1.10 (s, 3H), 1.06 (m, 13H), 0.98 (m, 6H), 0.79 (s, 3H).

### Example 12

### 2-(Dimethylamino)ethyl (2S,4aS,6aR,6bS,8aR,12aS,14bR)-11-cyano-2,4a,6a,6b,9,9,12a-heptamethyl-10-oxo-1,2,3,4,4a,5,6,6a,6b,7,8,8a,9,10,12a,14b-hexadecahydropicene-2-carboxylate

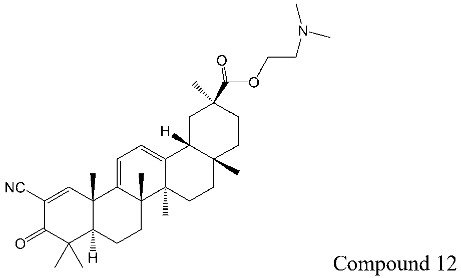

### Synthesis Procedure:

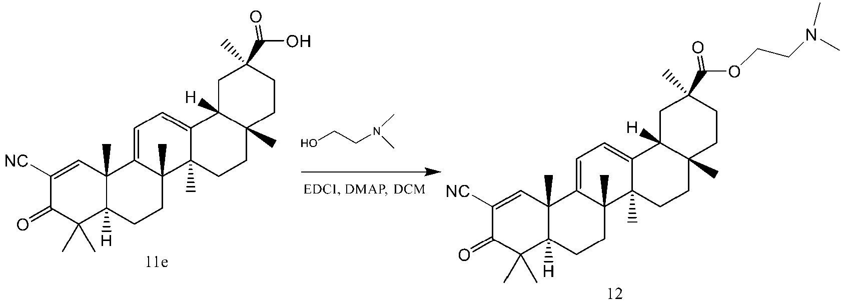

Preparation of Compound 12: The preparation procedure was performed with reference to Compound 11 in Example 11, except that diethylaminoethanol was replaced with dimethylaminoethanol, and the other steps were identical. The concentrate was purified by silica gel column chromatography (petroleum ether: tetrahydrofuran = 10:1) to afford Compound 12 (1.8 g, yield 39%). MS m/z (ESI): 547.37[M+H]⁺. ¹H-NMR (400 MHz, DMSO-d₆): δ 7.81 (s, 1H), 5.96 (d, 1H, J=5.61Hz), 5.62 (d, 1H, J=5.22Hz), 4.82 (m, 2H), 2.95 (m, 6H), 2.10 (m, 3H), 1.91 (m, 1H), 1.75 (m, 5H), 1.49 (m, 4H), 1.22 (m, 4H), 1.14 (s, 3H), 1.08 (s, 3H), 1.01 (m, 7H), 0.94 (m, 6H), 0.73 (s, 3H).

### Example 13

### 2-(Piperidin-1-yl)ethyl (2S,4aS,6aS,6bR,8aR,12aS,12bR,14bS)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate

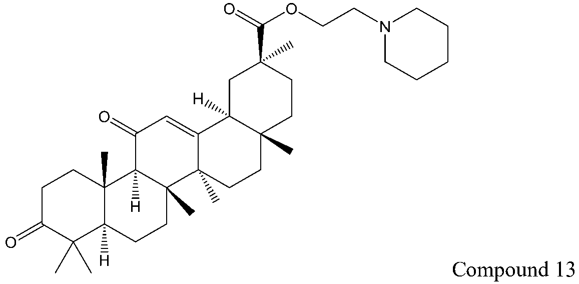

### Synthesis Procedure:

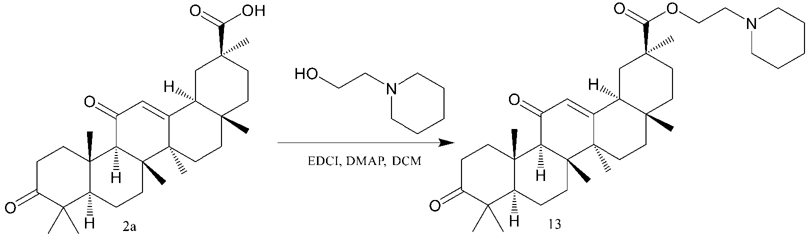

The preparation procedure was performed with reference to Compound 6 in Example 6, except that Intermediate 1a was replaced with Intermediate 2a, and the other steps were identical. MS m/z (ESI): 580.41[M+H]⁺. ¹H-NMR (400 MHz, DMSO-d6): δ 5.62 (s, 1H), 4.07 (m, 2H), 2.94 (m, 1H), 2.71(m, 2H), 2.46 (m, 4H), 2.32 (m, 1H), 2.14 (m, 2H), 1.95 (m, 5H), 1.42 (m, 8H), 1.35 (m, 8H), 1.31 (m, 2H), 1.16 (m, 4H), 1.03 (m, 14H), 0.75 (s, 3H).

### Example 14

### 2-Morpholinoethyl (2S,4aS,6aS,6bR,8aR,12aS,12bR,14bS)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate

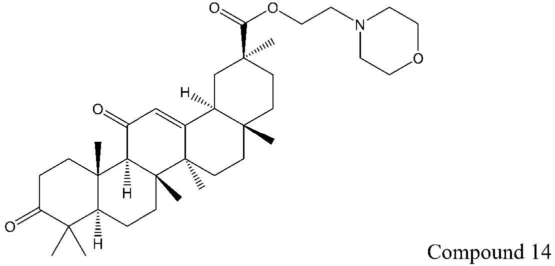

### Synthesis Procedure:

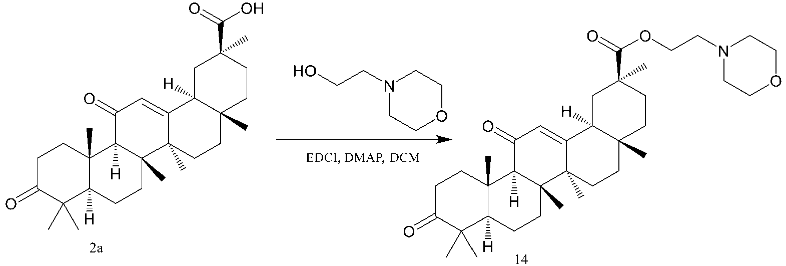

The preparation procedure was performed with reference to Compound 7 in Example 7, except that Intermediate 1a was replaced with Intermediate 2a, and the other steps were identical. MS m/z (ESI): 582.43[M+H]⁺. ¹H-NMR (400 MHz, DMSO-d₆): δ 5.98 (s, 1H), 4.13 (m, 2H), 3.57 (m, 4H), 3.03 (m, 2H), 2.82 (m, 1H), 2.47 (m, 4H), 2.38 (m, 1H), 2.21 (m, 2H), 1.89 (m, 5H), 1.62 (m, 4H), 1.43 (m, 6H), 1.31 (m, 2H), 1.25 (m, 4H), 1.05 (m, 14H), 0.89 (s, 3H).

### Example 15

### 2-(Pyrrolidin-1-yl)ethyl (2S,4aS,6aS,6bR,8aR,12aS,12bR,14bS)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate

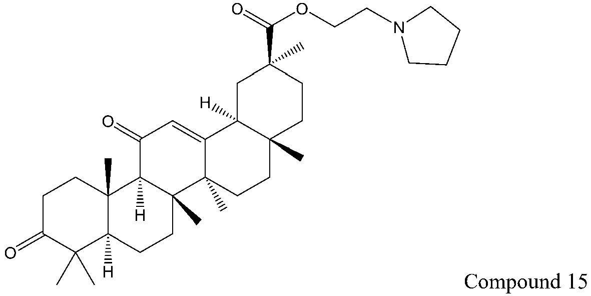

### Synthesis Procedure:

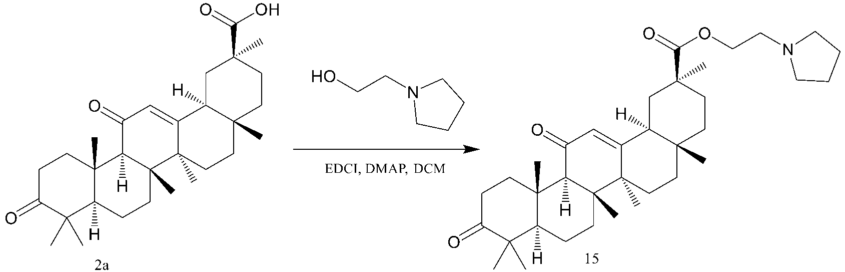

The preparation procedure was performed with reference to Compound 8 in Example 8, except that Intermediate 1a was replaced with Intermediate 2a, and the other steps were identical. MS m/z (ESI): 566.45[M+H]⁺. ¹H-NMR (400 MHz, DMSO-d6):δ 5.92 (s, 1H), 4.06 (m, 2H), 3.01 (m, 2H), 2.72 (m, 1H), 2.62 (m, 4H), 2.48 (m, 1H), 2.34 (m, 2H), 2.02 (m, 5H), 1.72 (m, 8H), 1.54 (m, 6H), 1.35 (m, 2H), 1.24 (m, 4H), 1.05 (m, 14H), 0.88 (s, 3H).

### Comparative Example 1

### 2-(Dimethylamino)ethyl (2S,4aS,6aS,6bR,8aR,12aS,12bR,14bR)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylate

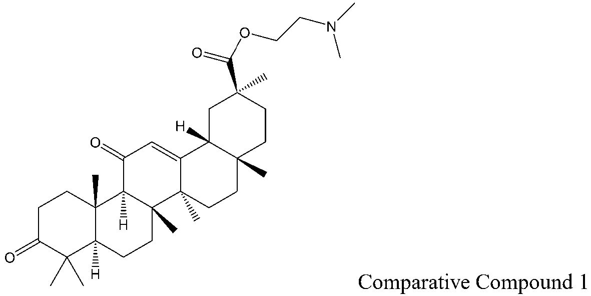

### Synthesis Procedure:

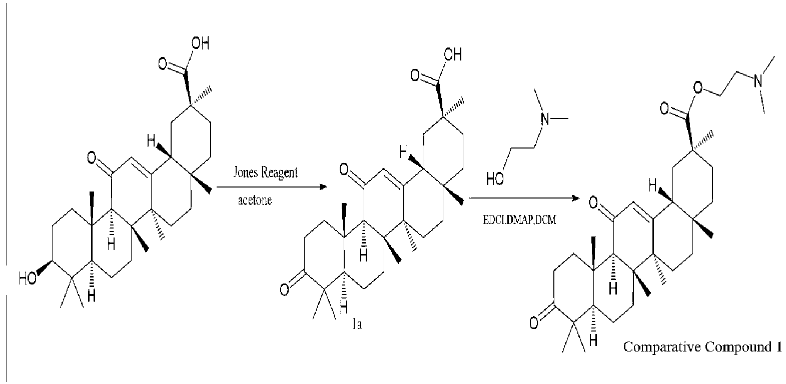

Preparation of Comparative Compound 1: The preparation procedure was performed with reference to Compound 1 in Example 1, except that diethylaminoethanol was replaced with dimethylaminoethanol, and the other steps were identical.

### Comparative Example 2

### 2-(Dimethylamino)ethyl (2S,4aS,6aS,6bR,8aR,10S,12aS,12bR,14bR)-10-hydroxy-2,4a,6a,6b,9,9,12a-heptamethyl-13-oxo-1,2,3,4,4a,5,6,6a,6b,7,8,8a,9,10,11,12,12a,12b,13,14b-icosahydropicene-2-carboxylate

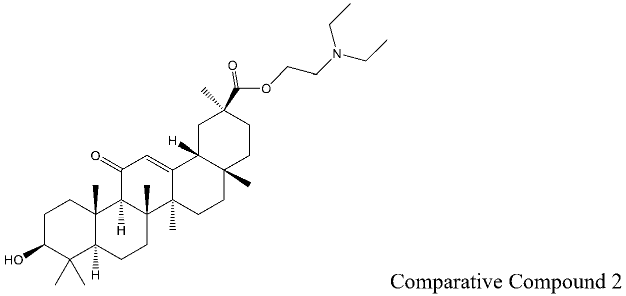

### Synthesis Procedure:

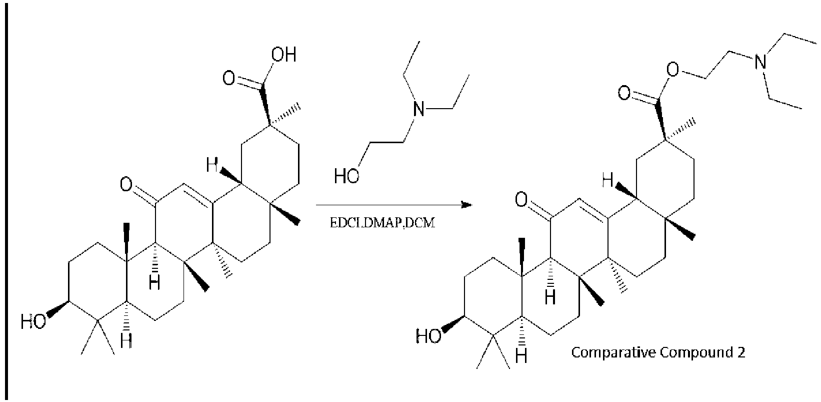

Preparation of Comparative Compound 2: The preparation procedure was performed with reference to Compound 1 in Example 1, except that Intermediate 1a was replaced with 18β-glycyrrhetinic acid, and the other steps were identical.

### Comparative Example 3

### (2S,4aS,6aS,6bR,8aR,12aS,12bR,14bR)-2,4a,6a,6b,9,9,12a-heptamethyl-10,13-dioxo-1,2,3,4,4a,5,6,6a,6b,7,8,8a,9,10,11,12,12a,12b,13,14b-icosahydropicene-2-carboxylic acid

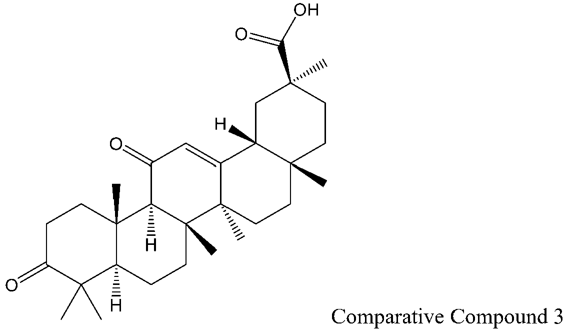

Intermediate 1a was used as Comparative Compound 3.

### Preparation of specific compounds having a structure as shown in formula (II) or a pharmaceutically acceptable salt thereof:

### Example 16

### (2S,4aS,6aS,6bR,8aR,12aS,12bR,14bR)-11-chloro-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylic acid

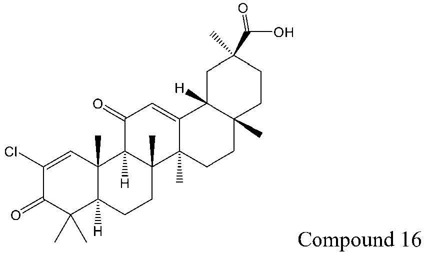

### Synthesis Procedure:

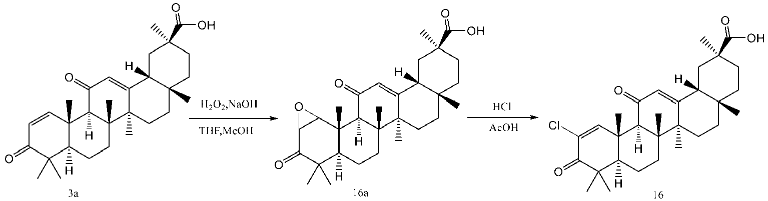

Preparation of Intermediate 16a: Intermediate 3a (10 g, 21 mmol) was dissolved in tetrahydrofuran (200 mL), and 2 M sodium hydroxide solution (100 mL) was slowly added dropwise. After the addition was complete, a solution of 30% hydrogen peroxide (25 mL) in methanol (100 mL) was added dropwise to the system. The reaction was carried out at room temperature and monitored by TLC until no Intermediate 3a remained. Most of the solvent was evaporated under reduced pressure. The mixture was adjusted to neutral with 5% hydrochloric acid solution, extracted with ethyl acetate, dried over anhydrous sodium sulfate, and concentrated in vacuo to afford Intermediate 16a (10 g, yield 97%). MS m/z (ESI): 483.31[M+H]⁺.

Preparation of Compound 16: Intermediate 16a (10 g, 21 mmol) was dissolved in 200 mL of glacial acetic acid, and concentrated hydrochloric acid (30 mL) was added dropwise. The reaction was carried out at room temperature and monitored by TLC until the raw material had completely reacted. The reaction mixture was poured into 1000 mL of water, and a large amount of white precipitate formed. The mixture was filtered, and the filter cake was washed several times with copious water to give a white solid. The crude product was purified by silica gel column chromatography (petroleum ether: tetrahydrofuran = 10:1) and concentrated to dryness to afford Compound 16 (5.1 g, yield 48%). MS m/z (ESI): 501.16[M+H]⁺. ¹H-NMR (400 MHz, DMSO-d₆): δ 12.19 (s, 1H), 7.92(s, 1H), 5.52 (s, 1H), 2.87(s, 1H), 2.13 (m, 2H), 1.81 (m, 6H), 1.55 (m, 2H), 1.38 (m, 11H), 1.07 (m,13H), 0.75 (s, 3H).

### Example 17

### (2S,4aS,6aS,6bR,8aR,12aS,12bR,14bS)-11-chloro-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylic acid

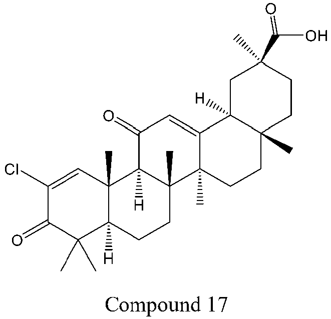

### Synthesis Procedure:

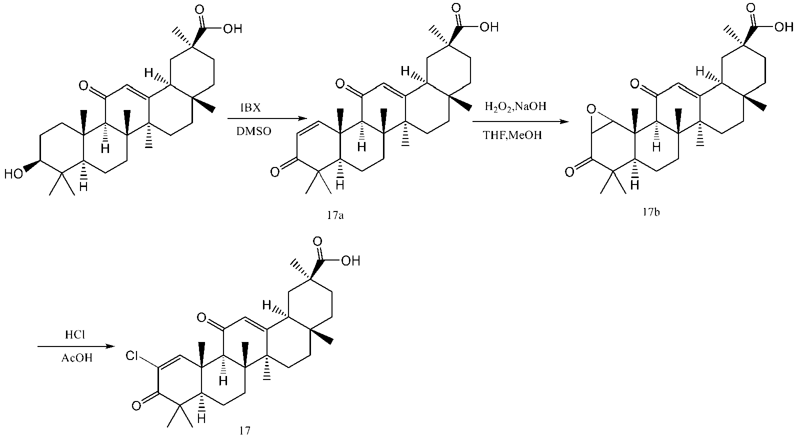

Preparation of Intermediate 17a: The preparation procedure was performed with reference to Intermediate 3a in Example 3, except that 18β-glycyrrhetinic acid was replaced with 18α-glycyrrhetinic acid, and the other steps were identical. MS m/z (ESI): 467.34[M+H]⁺.

Preparation of Intermediate 17b: The preparation procedure was performed with reference to Intermediate 16a in Example 16, except that Intermediate 3a was replaced with Intermediate 17a, and the other steps were identical. MS m/z (ESI): 483.31[M+H]⁺.

Preparation of Compound 17: The preparation procedure was performed with reference to Compound 16 in Example 16, except that Intermediate 16a was replaced with Intermediate 17b, and the other steps were identical. MS m/z (ESI): 501.28 [M+H]⁺. ¹H-NMR (400 MHz, DMSO-d₆): δ 12.19 (s, 1H), 7.92(s, 1H), 5.52 (s, 1H), 2.74(s, 1H), 2.05 (m, 2H), 1.76 (m, 6H), 1.53 (m, 2H), 1.36 (m, 11H), 1.07 (m,13H), 0.75 (s, 3H).

### Example 18

### (2S,4aS,6aS,6bR,8aR,12aS,12bR,14bR)-11-chloro-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylate triethanolamine salt

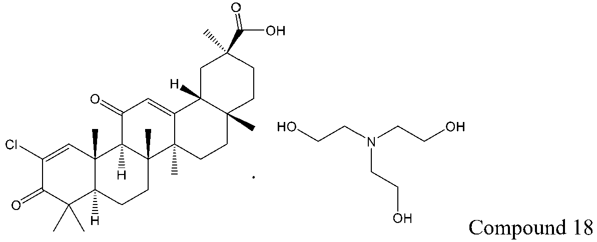

Preparation of Compound 18: Compound 16 was dissolved in dichloromethane, and an equivalent amount of triethanolamine was added. The reaction was carried out at room temperature for 24 h. Concentration afforded Compound 18. MS m/z (ESI): 501.31[M+H]⁺. ¹H NMR (400 MHz, DMSO-d₆): δ 12.19 (s, 1H), 7.92(s, 1H), 5.52 (s, 1H), 4.16 (s, 3H), 3.42 (m,6H), 2.87 (s, 1H), 2.57 (m,6H), 2.13 (m, 2H), 1.81 (m, 6H), 1.55 (m, 2H), 1.38 (m, 11H), 1.07 (m,13H), 0.75 (s, 3H).

### Example 19

### (2S,4aS,6aS,6bR,8aR,12aS,12bR,14bR)-11-bromo-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylic acid

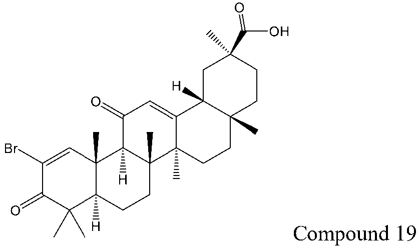

### Synthesis Procedure:

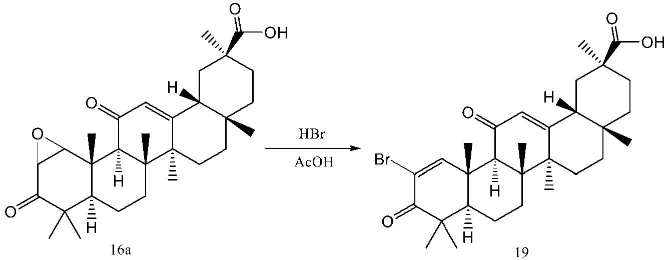

Preparation of Compound 19: The preparation procedure was performed with reference to Compound 16 in Example 16, except that concentrated hydrochloric acid was replaced with hydrobromic acid, and the other steps were identical. The white solid was purified by silica gel column chromatography (petroleum ether: tetrahydrofuran = 10:1) to afford Compound 19 (5.5 g, yield 49%). MS m/z (ESI): 545.23[M+H]⁺. ¹H-NMR (400 MHz, DMSO-d₆): δ 12.09 (s, 1H), 7.83(s, 1H), 5.32 (s, 1H), 2.72 (s, 1H), 2.01 (m, 2H), 1.89 (m, 6H), 1.65 (m, 2H), 1.43 (m, 11H), 1.21 (m,13H), 0.72 (s, 3H).

### Example 20

### (2S,4aS,6aS,6bR,8aR,12aS,12bR,14bS)-11-bromo-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylic acid

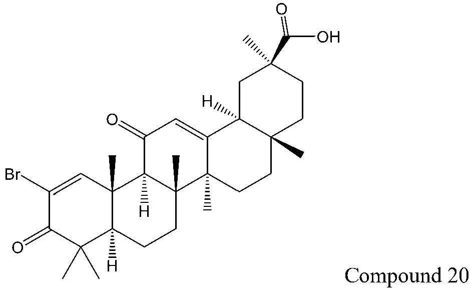

### Synthesis Procedure:

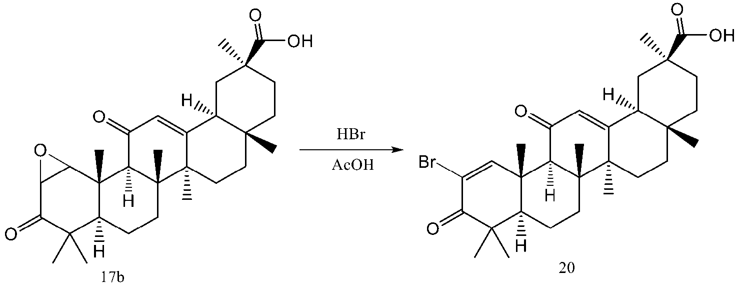

Preparation of Compound 20: The preparation procedure was performed with reference to Compound 17 in Example 17, except that concentrated hydrochloric acid was replaced with hydrobromic acid, and the other steps were identical. MS m/z (ESI): 545.36 [M+H]⁺. ¹H-NMR (400 MHz, DMSO-d6): δ 12.09 (s, 1H), 7.83(s, 1H), 5.32 (s, 1H), 2.65 (s, 1H), 2.12 (m, 2H), 1.74 (m, 6H), 1.63(m, 2H), 1.45 (m, 11H), 1.21 (m,13H), 0.72 (s, 3H).

### Comparative Example 4

### (2S,4aS,6aS,6bR,8aR,12aS,12bR,14bR)-11-iodo-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylic acid

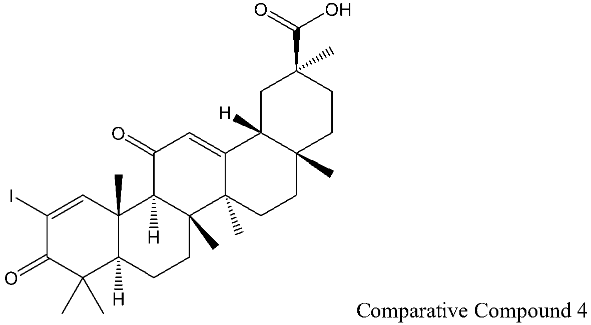

Intermediate 3b was used as Comparative Compound 4.

### Comparative Example 5

### (25,4a8,6aS,6bR,8aR,12a8,12bR,14bR)-11-fluoro-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylic acid

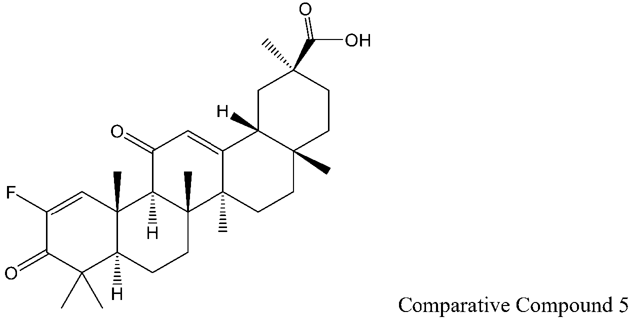

### Synthesis Procedure:

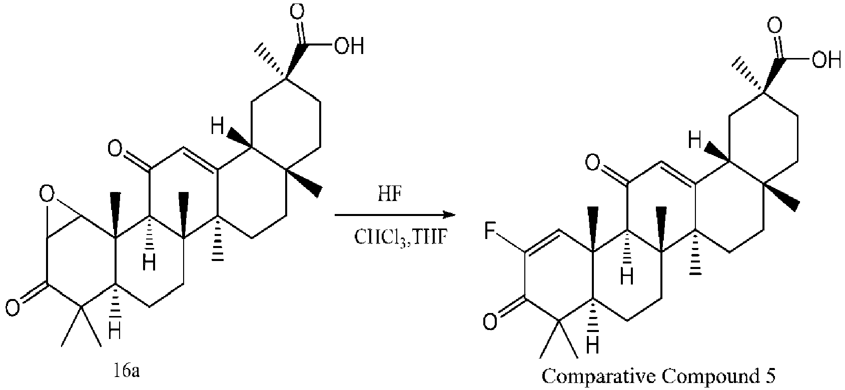

Preparation of Comparative Compound 5: Intermediate 16a (10 g, 21 mmol) was dissolved in 60 mL of chloroform, and a mixed solution of hydrofluoric acid (27 mL) in tetrahydrofuran (46 mL) and chloroform (27 mL) was slowly added dropwise to the system at 70°C. After the addition was complete, the reaction was carried out at room temperature and monitored by TLC until no Intermediate 16a remained. The reaction mixture was poured into 1000 mL of 5% potassium carbonate solution, extracted with chloroform, dried over anhydrous sodium sulfate, and concentrated in vacuo to give a crude product. The crude product was purified by silica gel column chromatography (petroleum ether: tetrahydrofuran = 10:1) to afford Comparative Compound 5 (2.1 g, yield 21%).

### Comparative Example 6

### (2S,4aS,6aS,6bR,8aR,12aR,12bR,14bR)-11-hydroxy-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylic acid

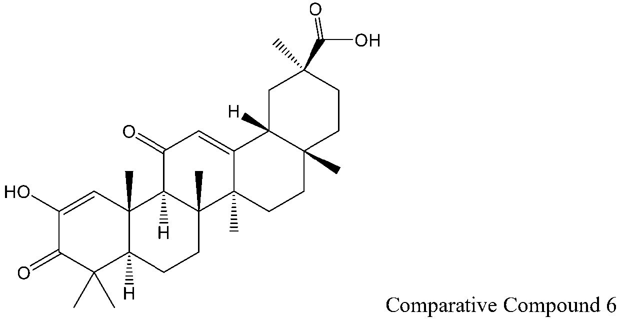

### Synthesis Procedure:

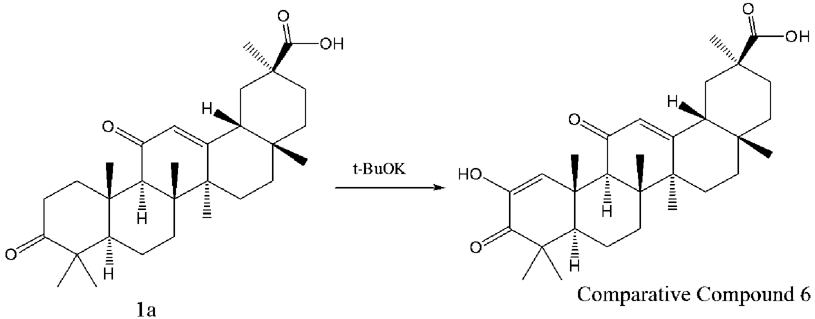

Preparation of Comparative Compound 6: Compound 1a (5 g, 10.7 mmol) was dissolved in 300 mL of tert-butanol, and potassium tert-butoxide (24 g, 213.3 mmol) was added. The reaction was carried out at 45°C and monitored by TLC until no Compound 1a remained. After cooling to room temperature, the mixture was adjusted to weakly acidic pH with 5 N hydrochloric acid solution, extracted with ethyl acetate, dried over anhydrous sodium sulfate, and concentrated in vacuo. The concentrate was purified by silica gel column chromatography (petroleum ether: tetrahydrofuran = 10:1) to afford Comparative Compound 6 (2 g, yield 39%).

### Comparative Example 7

### (2S,4aS,6aS,6bR,8aR,12aS,12bR,14bR)-11-cyano-2,4a,6a,6b,9,9,12a-heptamethyl-10,13-dioxo-1,2,3,4,4a,5,6,6a,6b,7,8,8a,9,10,12a,12b,13,14b-octadecahydropicene-2-carboxylic acid

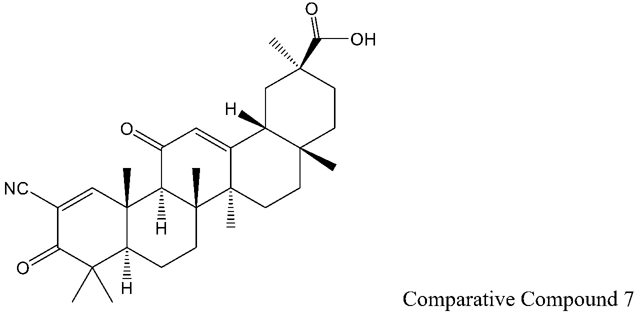

Intermediate 3c was used as Comparative Compound 7.

### Comparative Example 8

### (2S,4aS,6aR,6bS,8aR,12aS,14bR)-2,4a,6a,6b,9,9,12a-heptamethyl-10-oxo-1,2,3,4,4a,5,6,6a,6b,7,8,8a,9,10,11,12,12a,14b-octadecahydropicene-2-carboxylic acid

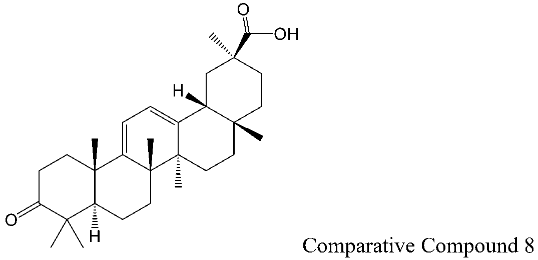

### Synthesis Procedure:

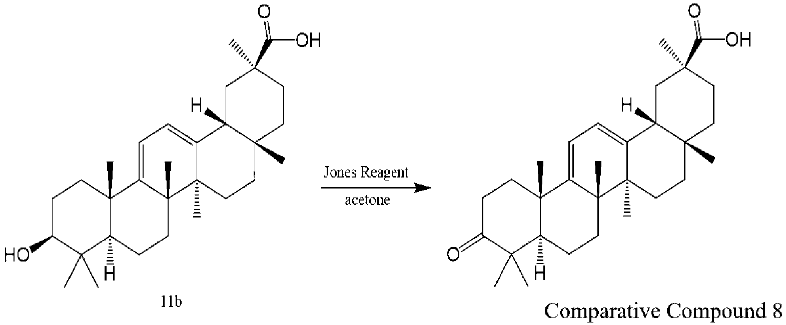

Preparation of Comparative Compound 8: The preparation procedure was performed with reference to Intermediate 1a in Example 1, except that 18β-glycyrrhetinic acid was replaced with Intermediate 11b, and the other steps were identical.

### Comparative Example 9

### (2S,4aS,6aR,6bS,8aR,12aS,14bR)-11-cyano-2,4a,6a,6b,9,9,12a-heptamethyl-10-oxo-1,2,3,4,4a,5,6,6a,6b,7,8,8a,9,10,12a,14b-hexadecahydropicene-2-carboxylic acid

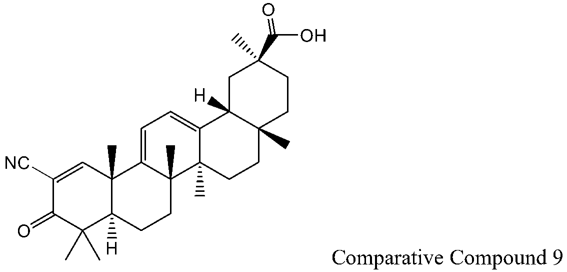

Intermediate 11e was used as Comparative Compound 9.

### Comparative Example 10

### (2S,4aS,6aR,6bS,8aR,12aR,14bR)-11-chloro-2,4a,6a,6b,9,9,12a-heptamethyl-10-oxo-1,2,3,4,4a,5,6,6a,6b,7,8,8a,9,10,12a,14b-hexadecahydropicene-2-carboxylic acid

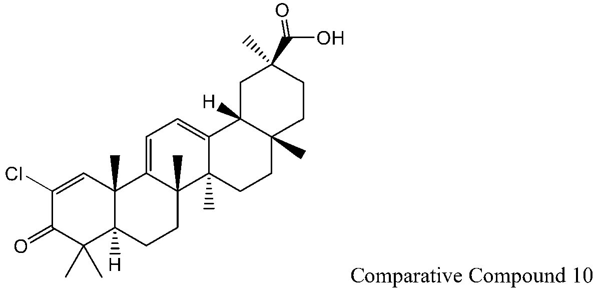

### Synthesis Procedure:

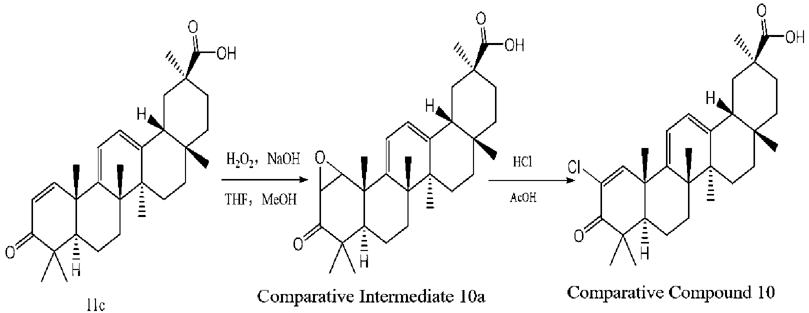

Preparation of Comparative Intermediate 10a: The preparation procedure was performed with reference to Intermediate 16a in Example 16, except that Intermediate 3a was replaced with Intermediate 11c, and the other steps were identical. MS m/z (ESI): 467.65[M+H]⁺.

Preparation of Comparative Compound 10: The preparation procedure was performed with reference to Compound 16 in Example 16, except that Intermediate 16a was replaced with Comparative Intermediate 10a, and the other steps were identical. MS m/z (ESI): 485.27[M+H]⁺. ¹H-NMR (400 MHz, DMSO-d6): δ12.13 (s, 1H), 7.31(s, 1H), 6.07 (s, 1H), 5.66 (s, 1H), 2.36(m, 2H), 2.14 (m, 2H), 1.98 (m, 2H), 1.80 (m, 1H), 1.52 (m, 3H), 1.44 (m, 8H), 1.11 (m, 1H), 1.06 (m, 3H), 1.02 (m, 6H), 0.96 (m, 6H), 0.83 (s, 3H).

### Preparation of specific compounds having a structure as shown in formula (III) or a pharmaceutically acceptable salt thereof:

### Example 21

### (2S,4aS,6aS,6bR,8aR,13aS,13bR,15bR)-11-(dimethylamino)-2,4a,6a,6b,9,9,13a-heptamethyl-14-oxo-1,2,3,4,4a,5,6,6a,6b,7,8,8a,9,13,13a,13b,14,15b-octadecahydropiceno[3,2-d]thiazole-2-carboxylic acid

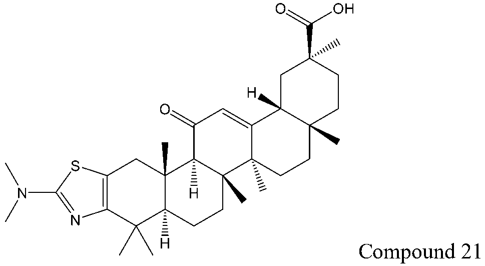

### Synthesis Procedure:

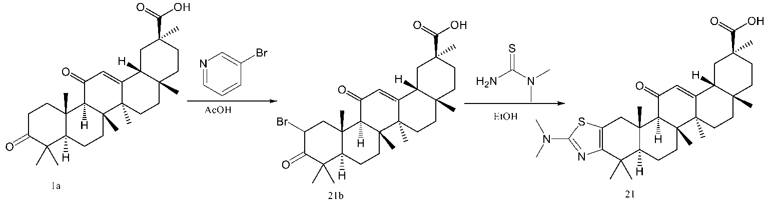

Preparation of Intermediate 21b: Intermediate 1a (18 g, 38 mmol) was dissolved in acetic acid (200 mL), and 3-bromopyridine (6.7 g, 42 mmol) was slowly added dropwise at room temperature. The reaction was carried out at room temperature and monitored by TLC until no Intermediate 1a remained. The solvent was evaporated under reduced pressure. The residue was added to ethyl acetate (200 mL), washed successively with saturated sodium carbonate solution (200 mL) and saturated sodium chloride solution (200 mL), dried over anhydrous sodium sulfate, and concentrated in vacuo to give a crude product. The crude product was purified by silica gel column chromatography (tetrahydrofuran) to afford Intermediate 21b (9 g, yield 43%). MS m/z (ESI): 547.24 [M+H]⁺.

Preparation of Compound 21: Intermediate 21b (9 g, 16 mmol) and 1,1-dimethylthiourea (1.7 g, 16 mmol) were dissolved in ethanol (100 mL). The reaction was carried out under reflux and monitored by TLC until no Intermediate 21b remained. The solvent was removed in vacuo to give a yellow solid. The solid was triturated three times with ethyl acetate (50 mL) to give a crude product. The crude product was purified by silica gel column chromatography (petroleum ether: tetrahydrofuran = 10:1) and concentrated to afford Compound 21 (4 g, 44%). MS m/z (ESI): 553.39[M+H]⁺. ¹H-NMR (400 MHz, DMSO-d6): δ 12.08 (s, 1H), 5.48 (s, 1H), 3.50(m, 1H), 3.21(s, 6H), 2.61(m, 1H), 2.10 (m, 2H), 1.72 (m, 6H), 1.60 (m, 8H), 1.44 (m, 3H), 1.37 (m, 14H), 1.09 (m, 2H), 0.77 (s, 3H).

### Example 22

### (2S,4aS,6aS,6bR,8aR,13aS,13bR,15bR)-11-(diethylamino)-2,4a,6a,6b,9,9,13a-heptamethyl-14-oxo-1,2,3,4,4a,5,6,6a,6b,7,8,8a,9,13,13a,13b,14,15b-octadecahydropiceno[3,2-d]thiazole-2-carboxylic acid

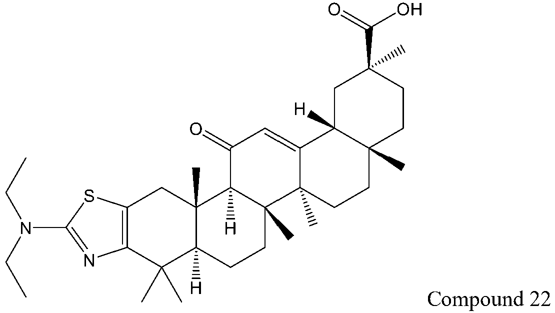

### Synthesis Procedure:

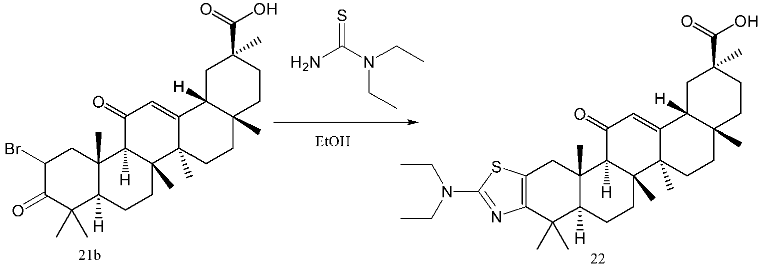

Preparation of Compound 22: Intermediate 21b (9 g, 16 mmol) and 1,1-diethylthiourea (2.2 g, 16 mmol) were dissolved in ethanol (100 mL). The reaction was carried out under reflux and monitored by TLC until no Intermediate 21b remained. The solvent was removed in vacuo to give a yellow solid. The solid was triturated three times with ethyl acetate (50 mL) to give a crude product. The crude product was purified by silica gel column chromatography (petroleum ether: tetrahydrofuran = 10:1) and concentrated to afford Compound 22 (3.5 g, 37%). MS m/z (ESI): 581.35[M+H]⁺. ¹H-NMR (400 MHz, DMSO-d6): δ 12.08 (s, 1H), 5.77 (s, 1H), 3.46(m, 1H), 3.12(m, 4H), 2.54(m, 1H), 2.13 (m, 2H), 1.67 (m, 6H), 1.58 (m, 8H), 1.37 (m, 3H), 1.29 (m, 14H), 1.07 (m, 8H), 0.72 (s, 3H).

### Example 23

### (2S,4aS,6aS,6bR,8aR,13aS,13bR,15bR)-11-(dipropylamino)-2,4a,6a,6b,9,9,13a-heptamethyl-14-oxo-1,2,3,4,4a,5,6,6a,6b,7,8,8a,9,13,13a,13b,14,15b-octadecahydropiceno[3,2-d]thiazole-2-carboxylic acid

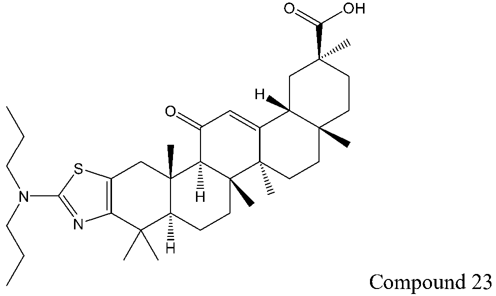

### Synthesis Procedure:

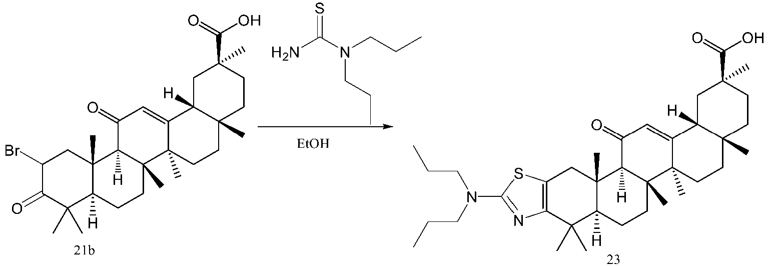

Preparation of Compound 23: Intermediate 21b (9 g, 16 mmol) and 1,1-dipropylthiourea (2.6g, 16 mmol) were dissolved in ethanol (100 mL). The reaction was carried out under reflux and monitored by TLC until no Intermediate 21b remained. The solvent was removed in vacuo to give a yellow solid. The solid was triturated three times with ethyl acetate (50 mL) to give a crude product. The crude product was purified by silica gel column chromatography (petroleum ether: tetrahydrofuran = 10:1) and concentrated to afford Compound 23 (3.8 g, 38%). MS m/z (ESI): 609.40[M+H]⁺. ¹H-NMR (400 MHz, DMSO-d6): δ 12.08 (s, 1H), 5.75 (s, 1H), 3.52(m, 1H), 3.49(m, 4H), 2.45(m, 1H), 2.17 (m, 2H), 1.76 (m, 6H), 1.57 (m, 12H), 1.32 (m, 3H), 1.25 (m, 14H), 1.06 (m, 2H), 0.87 (m, 6H), 0.70 (s, 3H).

### Example 24

### (2S,4aS,6aS,6bR,8aR,13aS,13bR,15bR)-11-(dimethylamino)-2,4a,6a,6b,9,9,13a-heptamethyl-14-oxo-1,2,3,4,4a,5,6,6a,6b,7,8,8a,9,13,13a,13b,14,15b-octadecahydropiceno[3,2-d]thiazole-2-carboxylic acid hydrochloride

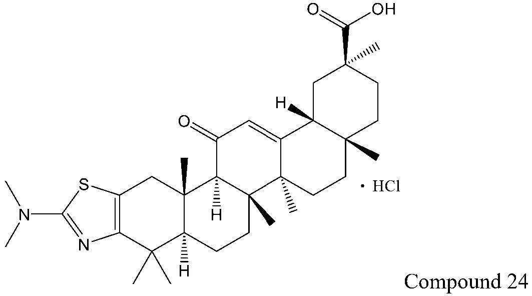

### Synthesis Procedure:

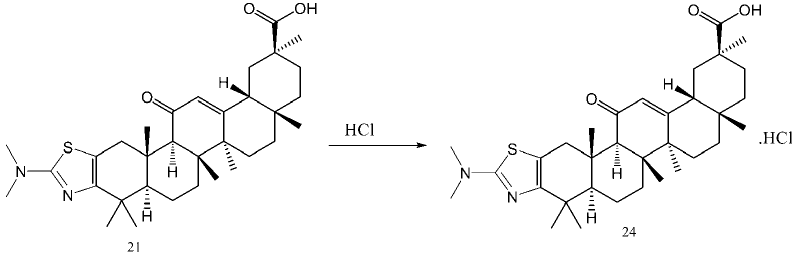

Preparation of Compound 24: Compound 21 (2 g, 3.2 mmol) was dissolved in 20 mL of dichloromethane, and a solution of hydrochloric acid in isopropanol was added dropwise. The reaction was carried out at room temperature for 24 h. Concentration afforded Compound 24 (1.9 g, yield 89%). MS m/z (ESI): 553.39[M+H]⁺.

### Example 25

### (2S,4aS,6aS,6bR,8aR,13aS,13bR,15bR)-11-(diethylamino)-2,4a,6a,6b,9,9,13a-heptamethyl-14-oxo-1,2,3,4,4a,5,6,6a,6b,7,8,8a,9,13,13a,13b,14,15b-octadecahydropiceno[3,2-d]thiazole-2-carboxylic acid hydrochloride

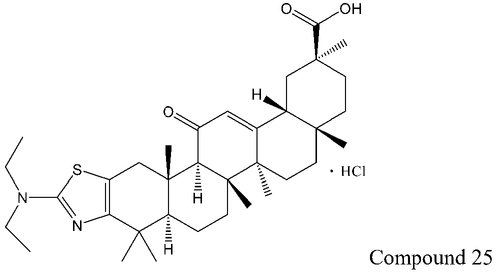

### Synthesis Procedure:

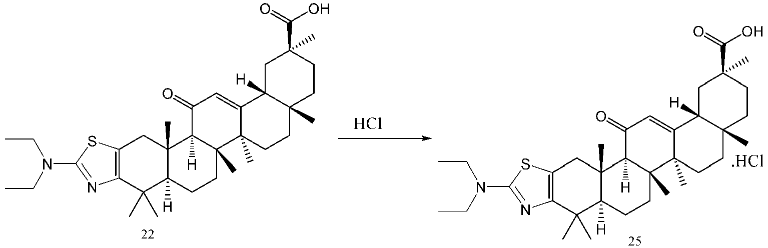

Preparation of Compound 25: Compound 22 (2 g, 3.4 mmol) was dissolved in 20 mL of dichloromethane, and a solution of hydrochloric acid in isopropanol was added dropwise. The reaction was carried out at room temperature for 24 h. Concentration afforded Compound 25 (1.8 g, yield 86%). MS m/z (ESI): 581.35[M+H]⁺.

### Example 26

### (2S,4aS,6aS,6bR,8aR,13aS,13bR,15bR)-11-(dipropylamino)-2,4a,6a,6b,9,9,13a-heptamethyl-14-oxo-1,2,3,4,4a,5,6,6a,6b,7,8,8a,9,13,13a,13b,14,15b-octadecahydropiceno[3,2-d]thiazole-2-carboxylic acid hydrochloride

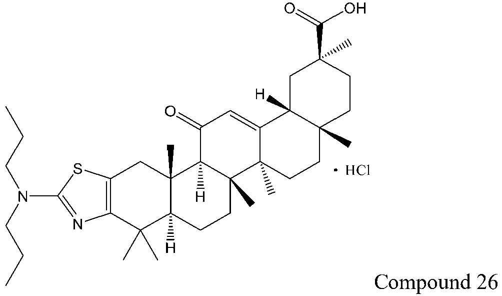

### Synthesis Procedure:

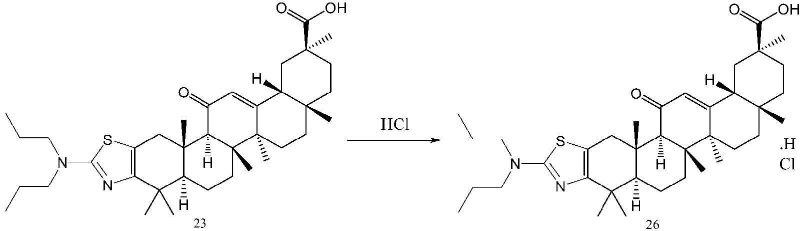

Preparation of Compound 26: Compound 23 (2 g, 3.3 mmol) was dissolved in 20 mL of dichloromethane, and a solution of hydrochloric acid in isopropanol was added dropwise. The reaction was carried out at room temperature for 24 h. Concentration afforded Compound 26 (1.9 g, yield 90%). MS m/z (ESI): 609.40[M+H]⁺.

### Example 27

### 2-(Diethylamino)ethyl (2S,4aS,6aS,6bR,8aR,13aS,13bR,15bR)-11-(dimethylamino)-2,4a,6a,6b,9,9,13a-heptamethyl-14-oxo-1,2,3,4,4a,5,6,6a,6b,7,8,8a,9,13,13a,13b,14,15b-octadecahydropiceno[3,2-d]thiazole-2-carboxylate

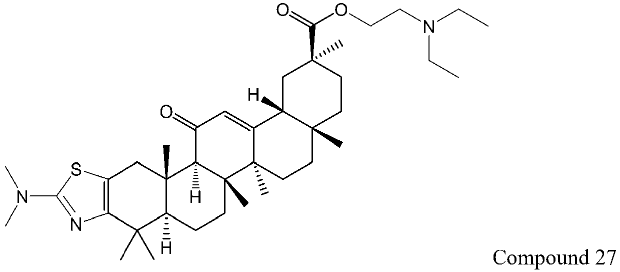

### Synthesis Procedure:

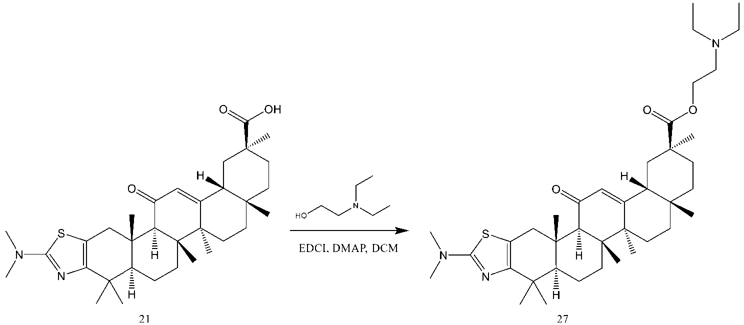

Preparation of Compound 27: The preparation procedure was performed with reference to Compound 1 in Example 1, except that Intermediate 1a was replaced with Compound 21, and the other steps were identical. MS m/z (ESI): 652.45 [M+H]⁺. ¹H-NMR (400 MHz, DMSO-d6): δ 5.72 (s, 1H), 4.27 (m, 2H), 3.58 (m, 1H), 3.10 (s,6H), 2.72 (m, 2H), 2.61 (m, 2H), 2.53 (m, 3H), 2.23 (m, 2H), 1.84 (m, 6H), 1.67 (m, 8H), 1.51 (m, 3H), 1.41 (m, 14H), 1.16 (m, 8H), 0.73 (m, 3H).

### Experimental Example 1: Anti-Inflammatory Activity Assay in Cells

1.1 Cells: RAW264.7 cells, purchased from PROCELL, lot No.: JYBR1POD9R.
1.2 Reagents: Lipopolysaccharide (LPS), purchased from SIGMA; DMEM cell culture medium, purchased from GIBCO; Mouse IL-1β ELISA Kit, purchased from Ruixin Bio.

### 1.3 Methods

RAW264.7 cells were seeded into a 96-well plate at 1×10⁵ cells/well, with 100 µL of cell suspension added to each well. 50 µL of drugs at corresponding gradient concentrations (0.00128, 0.0064, 0.032, 0.16, 0.8, 4, 20, and 100 µM, respectively) were added to the wells of the test groups (Compounds 1 to 27) and the control drug groups (glycyrrhetinic acid and Comparative Compounds 1 to 10). The corresponding volume of culture medium was added to the wells of the blank control group and the model group. After 1 h of incubation, 50 µL of LPS (final LPS concentration of 100 ng/mL) was added to the wells of the model group, the control drug groups, and the test groups. After incubation in an incubator at 37°C and 5% CO₂ for 24 h, the supernatant was collected by centrifugation at 1000 rpm for 5 min. The IL-1β content was determined according to the instructions of the kit, and the IC₅₀ values were calculated.

### 1.4 Experimental Results

| Group | IC₅₀ (µM) | Group | IC₅₀ (µM) |
|---|---|---|---|
| Glycyrrhetinic acid | D | Compound 16 | A |
| Compound 1 | A | Compound 17 | A |
| Compound 2 | A | Compound 18 | A |
| Compound 3 | A | Compound 19 | C |
| Compound 4 | B | Compound 20 | C |
| Compound 5 | B | Comparative Compound 4 | D |
| Compound 6 | B | Comparative Compound 5 | D |
| Compound 7. | B | Comparative Compound 6 | D |
| Compound 8 | B | Comparative Compound 7 | D |
| Compound 9 | A | Comparative Compound 8 | D |
| Compound 10 | B | Comparative Compound 9 | D |
| Compound 11 | C | Comparative Compound 10 | D |
| Compound 12 | C | Compound 21 | B |
| Compound 13 | B | Compound 22 | B |
| Compound 14 | B | Compound 23 | B |
| Compound 15 | B | Compound 24 | B |
| Comparative Compound 1 | D | Compound 25 | B |
| Comparative Compound 2 | D | Compound 26 | B |
| Comparative Compound 3 | D | Compound 27 | B |

| | | | |
|---|---|---|---|
| Note: A: less than or equal to 0.02 µM; B: greater than 0.02 µM and less than or equal to 0.1 µM; C: greater than 0.1 µM and less than or equal to 1 µM; D: greater than 1 µM. | | | |

### Experimental Example 2: Inhibitory Activity Assay against Ear Swelling in Guinea Pigs

2.1 Animals: Ordinary grade guinea pigs, weighing about 300 g.
2.2 Reagents: Inflammatory agent made from 80% dimethyl sulfoxide (DMSO) and 20% absolute ethanol; DMSO; absolute ethanol, analytical grade.

### 2.3 Methods

Ordinary grade guinea pigs were randomly divided into groups, with 10 animals in each group, half male and half female. The inner and outer sides of the left and right ears of the guinea pigs were shaved, and 4 points were marked on the medial side of the left ear margin. The thickness of these 4 points on the auricle of the guinea pigs was measured using a vernier caliper, and the average value was obtained as the baseline before administration. The corresponding drugs were applied dropwise to the auricles of the guinea pigs in each test group (Compound 1, Compound 2, Compound 3, Compound 9, Compound 16, Compound 17, Compound 18, Compound 21) and the glycyrrhetinic acid group, with 200 µL per ear. The blank control group and the model group were treated with the corresponding volume of absolute ethanol only. The positive control hydrocortisone butyrate group was treated with an equal volume of hydrocortisone butyrate cream. After 30 min of administration, the drugs were wiped off. The animals in the model group and the test groups were treated with the inflammatory agent on both sides of the left auricle (50 µL per ear). The blank control group was treated with the corresponding volume of normal saline only. At 45 min after modeling, the thickness of these 4 points was measured using a vernier caliper, and the average value was obtained. The difference was calculated as the ear swelling value of the guinea pigs. Inhibition rate of swelling value = (Swelling value of the model group - Swelling value of the sample group) / Swelling value of model group × 100%, wherein the sample group includes each test group, glycyrrhetinic acid group, and positive control hydrocortisone butyrate group.

### 2.4 Formulation Sources

Test compounds and glycyrrhetinic acid: Dissolved in absolute ethanol to prepare a solution with a concentration of 40 mg/mL.
Positive control: Commercially available hydrocortisone butyrate cream (from Hubei Heng'an Fulin Pharmaceutical Co., Ltd., specification: 1 mg/g).

### 2.5 Experimental Results

| Group | Ear swelling inhibition rate (%) |
|---|---|
| Hydrocortisone butyrate | A |
| Glycyrrhetinic acid | C |
| Compound 1 | A |
| Compound 2 | A |
| Compound 3 | B |
| Compound 9 | B |
| Compound 16 | A |
| Compound 17 | A |
| Compound 18 | B |
| Compound 21 | A |

| | |
|---|---|
| Note: A: greater than 70%; B: greater than 40% and less than or equal to 70%; C: 40% or less. | |

It can be seen that the compounds of the present disclosure exhibit excellent anti-inflammatory effects on subjects with inflammatory diseases.

### Experimental Example 3: Inhibitory Activity Assay against Ear Swelling in ICR Mice

### 3.1 Animals:

ICR mice, male, 20±2 g.
3.2 Reagents: Inflammatory agent (phorbol ester (TPA)).

### 3.3 Methods

ICR mice were randomly divided into groups, with 8 mice in each group. The thickness of the right ear of each animal was measured using a micrometer. Subsequently, except for the blank control group, all mice in other groups were administered 20 µL of 0.03% TPA on both the inner and outer sides of the right ear (10 µL per side) to induce inflammation. The mice were dosed once at 1 h before modeling and 1 h after modeling, respectively. The test groups (Compound 1, Compound 16) received a dose volume of 50 µL each time. The blank control group and the model group (with matrix) were treated with the corresponding volumes of blank matrix (pimecrolimus cream matrix). The positive control group was treated with an equal volume of pimecrolimus cream or compound dexamethasone acetate cream. Before modeling, the ears should be cleaned prior to TPA application. Ear thickness measurement: The thickness of the right ear was measured at 0 h, 2 h, 4 h, and 6 h after TPA induction.

### 3.4 Formulation Sources

Test compounds: The formulations were prepared with reference to pimecrolimus cream (from MEDA Manufacturing) (test compound concentration: 3%).
Positive control: Commercially available compound dexamethasone acetate cream (from China Resources Sanjiu Medical & Pharmaceutical Co., Ltd., specification: 0.75 mg/g); commercially available pimecrolimus cream (from MEDA Manufacturing, specification: 10 mg/g).

### 3.5 Experimental Results

| No. | Group | Thickness (mm) | | | |
|---|---|---|---|---|---|
| | | 0 h | 2 h | 4 h | 6 h |
| 1 | Blank control | 0.212+0.019 | 0.218+0.013 | 0.217±0.012 | 0.22+0.015 |
| 2 | Model (without matrix) | 0.215+0.007 | 0.309±0.042^{###} | 0.511±0.051^{###} | 0.61±0.031^{###} |
| 3 | Model (with matrix) | 0.214+0.008 | 0.285±0.015^{###} | 0.459±0.046^{###} | 0.463±0.055^{###} |
| 4 | Pimecrolimus | 0.211+0.008 | 0.268+0.035 | 0.32±0.035*** | 0.339±0.046*** |
| 5 | Dexamethasone | 0.216+0.008 | 0.266+0.029 | 0.268±0.046*** | 0.299±0.026*** |
| 6 | Compound 1 | 0.213+0.01 | 0.264+0.027 | 0.300±0.043*** | 0.293±0.066*** |
| 7 | Compound 16 | 0.215+0.008 | 0.262+0.035 | 0.303±0.032*** | 0.352±0.033*** |

| | | | | | |
|---|---|---|---|---|---|
| Note: Compared with the blank control group, ^{##}P < 0.01, ^{###}P < 0.001; compared with the model (with matrix) group, *P < 0.05, **P < 0.01, ***P < 0.001. | | | | | |

The experimental results showed that Compounds 1 and 16 of the present disclosure exhibited significant inhibitory activity (P < 0.001) in the mouse ear swelling inflammation model induced by phorbol ester, which was significantly superior to that of the model group.

Compounds 2, 3, 9, 17, 18, and 21 of the present disclosure also exhibited significant inhibitory activity in the mouse ear swelling inflammation model induced by phorbol ester, as determined by the methods described above.

### Experimental Example 4: Atopic Dermatitis Assay

4.1 Animals: BALB/c mice, female, 20+2 g.
4.2 Reagent: 2,4-Dinitrofluorobenzene (DNFB).

### 4.3 Methods

The backs of BALB/c mice were shaved. Except for the blank control group, the mice were topically treated with DNFB solution (solvent: mixture of acetone and olive oil, volume ratio 4:1) for sensitization and challenge. No treatment was administered on days 3 to 6. From day 7, the solution was applied once every 2 days for a total of 10 applications, and modeling was completed after the 10th application. The model animals were evenly divided into groups according to the observation scores of skin lesions, with 8 mice in each group. Subsequently, the test groups (Compound 1, Compound 16) received the corresponding drugs at 150 µL per time, twice daily. The model group received the corresponding volume of blank matrix (pimecrolimus cream matrix) at the same dosing frequency. The observation scores of skin lesions were recorded on days 3, 6, and 10 (six items were scored: erythema/darkening, edema/papules, exudation/crusting, erosion, lichenification/prurigo, and dryness, and the total score was the sum of the six individual scores; scoring was performed according to the SCORAD scoring criteria: scores 0 to 3, where score 0: none; score 1: mild; score 2: moderate; score 3: severe). Body weights were measured on days 0, 3, 6, and 10 after modeling.

### 4.4 Formulation Sources

Test compounds: The formulations were prepared with reference to pimecrolimus cream (test compound concentration: 3%).

### 4.5 Experimental Results

### 4.5.1 SCORAD Score

| No. | Group | Score | | | |
|---|---|---|---|---|---|
| | | Day 0 | Day 3 | Day 6 | Day 10 |
| 1 | Blank control | 0+0 | 0+0 | 0+0 | 0+0 |
| 2 | Model (with matrix) | 16.69±0.46^{###} | 14.13±0.44^{###} | 11.14±0.9^{###} | 10.38±0.52^{###} |
| 3 | Compound 1 | 16.56+0.50 | 10.31±0.88*** | 10.31±1.19** | 7.31±0.96*** |
| 4 | Compound 16 | 16.69+0.46 | 9.25±1.65*** | 7.56+1.47*** | 3.63±0.95*** |

| | | | | | |
|---|---|---|---|---|---|
| Note: Compared with the blank control group, ^{##}P < 0.01, ^{###}P < 0.001; compared with the model (with matrix) group, *P < 0.05, **P < 0.01, ***P < 0.001. | | | | | |

The experimental results showed that, compared with the model group, Compounds 1 and 16 of the present disclosure could significantly reduce the SCORAD score (P < 0.001) in the mouse atopic dermatitis assay.

Compounds 2, 3, 9, 17, 18, and 21 of the present disclosure also could significantly reduce the SCORAD score in the mouse atopic dermatitis model, as determined by the methods described above.

### 4.5.2 Body Weight

| No. | Group | Body weight of animals (g) | | | |
|---|---|---|---|---|---|
| | | Day 0 | Day 3 | Day 6 | Day 10 |
| 1 | Blank control | 19.78+0.56 | 20.28+0.83 | 19.73+0.99 | 19.71±0.8 |
| 2 | Model (with matrix) | 19.96+1.04 | 20+1.10 | 19.53+1.02 | 19.74+1.17 |
| 3 | Compound 1 | 20.01±0.84 | 19.45±0.99 | 19.05+1.16 | 18.86+0.95 |
| 4 | Compound 16 | 20.25+1.34 | 19.91+1.41 | 19.88+1.14 | 20.55+1.23 |

The experimental results showed that Compounds 1 and 16 of the present disclosure had no significant effect on the body weight of mice, indicating favorable safety.

Compounds 2, 3, 9, 17, 18, and 21 of the present disclosure also had no significant effect on body weight in the mouse atopic dermatitis model, indicating favorable safety, as determined by the methods described above.

### Experimental Example 5: Radiation Dermatitis Assay

5.1 Animals: SD rats, SPF grade, male, 230-250 g.
5.2 Equipment: RS 2000·Pro 225 Rad Source X-ray Irradiator; SpectraMax i3 Multifunctional Microplate Reader.

### 5.3 Methods

Eighty SD rats were randomly divided into groups, with 10 rats in each group. Except for the blank control group, all rats were subjected to modeling using a Rad Source X-ray irradiator. The hair on the irradiation site was removed 24 hours before irradiation. On the day of irradiation, the rats were anesthetized and received a single X-ray irradiation on the posterior buttock at a dose of 25 Gy, a dose rate of 4 Gy/min, and an irradiation area of 2×2 cm². On the second day after irradiation, the test groups (Compound 1, Compound 16, Compound 21) received the corresponding drugs, 100 mg per time, once daily for 28 consecutive days. The blank control group and the model (with matrix) group received the corresponding weight of blank matrix (triethanolamine cream matrix). The positive control group was treated with the corresponding weight of triethanolamine cream or hydrocortisone butyrate cream. At the end of administration, the damaged skin was scored according to erythema/darkening, edema/papules, exudation/crusting, and erosion, with reference to the scoring criteria for atopic dermatitis. After scoring, skin from the damaged site was collected and homogenized, and the content of inflammatory cells in the skin was detected using an ELISA kit. Additional skin was collected, fixed, and then stained with HE, and the number of inflammatory cells was observed.

### 5.4 Formulation Sources

Test compounds: The formulations were prepared with reference to triethanolamine cream (test compound concentration: 0.5%).
Positive controls: Commercially available triethanolamine cream (from JOHNSON & JOHNSON SANTE BEAUTE FRANCE, specification: 0.67%); hydrocortisone butyrate cream (from Hubei Heng'an Fulin Pharmaceutical Co., Ltd., specification: 1 mg/g).

### 5.5 Experimental Results

### 5.5.1 Number of Inflammatory Cells

| No. | Group | Number of inflammatory cells |
|---|---|---|
| 1 | Blank control | 43.3±30.9 |
| 2 | Model (without matrix) | 260.7±108.6^{###} |
| 3 | Model (with matrix) | 312.0±65.3^{###} |
| 4 | Triethanolamine | 257.7±75.4* |
| 5 | Hydrocortisone butyrate | 191.5±68.1** |
| 6 | Compound 1 | 186.6+78.6*** |
| 7 | Compound 16 | 182.6±73.3*** |
| 8 | Compound 21 | 188.6±56.3*** |

| | | |
|---|---|---|
| Note: Compared with the blank control group, ^{##}P < 0.01, ^{###}P < 0.001; compared with the model (with matrix) group, *P < 0.05, **P < 0.01, ***P < 0.001. | | |

The experimental results showed that Compounds 1, 16, and 21 of the present disclosure exhibited significant anti-inflammatory effects in the rat radiation dermatitis model and could significantly reduce the number of inflammatory cells (P < 0.001).

Compounds 2, 3, 9, 17, and 18 of the present disclosure also exhibited significant anti-inflammatory effects in the rat radiation dermatitis model and could significantly reduce the number of inflammatory cells, as determined by the methods described above.

In addition, the experimental results showed that Compounds 1, 2, 3, 9, 16, 17, 18, and 21 of the present disclosure exhibited significant anti-inflammatory effects in the rat radiation dermatitis model, and could significantly reduce the release of the inflammatory cytokine TNF-α and the content of the cytokine TGF-β1, as determined by the methods described above.

### 5.5.2 Skin Lesion Score of Radiation Dermatitis

| No. | Group | Score |
|---|---|---|
| 1 | Blank control | 0.0+0.0 |
| 2 | Model (without matrix) | 6.1±0.3^{###} |
| 3 | Model (with matrix) | 5.9±0.3^{###} |
| 4 | Triethanolamine | 5.3±0.7* |
| 5 | Hydrocortisone butyrate | 5.5+0.8* |
| 6 | Compound 1 | 4.8±0.7** |
| 7 | Compound 16 | 4.7±0.9*** |
| 8 | Compound 21 | 4.8±0.7** |

| | | |
|---|---|---|
| Note: Compared with the blank control group, ^{##}P < 0.01, ^{###}P < 0.001; compared with the model (with matrix) group, *P < 0.05, **P < 0.01, ***P < 0.001. | | |

The experimental results showed that Compounds 1, 16, and 21 of the present disclosure exhibited significant anti-inflammatory effects in the rat radiation dermatitis model and could significantly reduce the skin lesion score (P < 0.001, P < 0.01).

Compounds 2, 3, 9, 17, and 18 of the present disclosure also exhibited significant anti-inflammatory effects in the rat radiation dermatitis model and could significantly reduce the skin lesion score, as determined by the methods described above.

In addition, the experimental results showed that Compounds 1, 2, 3, 9, 16, 17, 18, and 21 of the present disclosure exhibited significant anti-inflammatory effects in the rat radiation dermatitis model and could significantly reduce the skin lesion area, as determined by the methods described above.

Meanwhile, pathological observation of rat skin showed that Compounds 1, 2, 3, 9, 16, 17, 18, and 21 of the present disclosure significantly increased the number of capillaries and the amount of new granulation tissue in the dermis, indicating that the compounds of the present disclosure can promote epidermal regeneration.

### Experimental Example 6: Effect of Compounds on Chloroquine-Induced Pruritus in Mice

6.1 Animals: Balb/c mice, female.
6.2 Reagents: Chloroquine; normal saline.

### 6.3 Methods

After 3 days of adaptive feeding, 50 female Balb/c mice were randomly divided into groups, with 10 mice in each group. The hair on a 2×3 cm² area of the back of each mouse was completely removed using depilatory cream. Except for the blank control group, the test groups (Compound 1, Compound 16) and the model group were treated with the corresponding drugs or matrix (pimecrolimus cream matrix) at the depilated site, 100 mg per time, once daily for 3 consecutive days. The positive control group was treated with the corresponding weight of pimecrolimus cream. Thirty minutes after the last administration, chloroquine (40 mg/kg) was injected subcutaneously. The blank control group was injected subcutaneously with the corresponding volume of normal saline only. The number of scratching bouts within 20 min after chloroquine injection was recorded.

### 6.4 Formulation Sources

Test compounds: The formulations were prepared with reference to pimecrolimus cream (test compound concentration: 3%).
Positive control: Commercially available pimecrolimus cream (from MEDA Manufacturing, specification: 10 mg/g).

### 6.5 Experimental Results

| No. | Group | Number of scratching bouts | | |
|---|---|---|---|---|
| | | **0-10** min | 10-20 min | 0-20 min |
| 1 | Blank control | 9+3 | 7+4 | 17+6 |
| 2 | Model (with matrix) | 23±11^{##} | 27±10^{###} | 50±17^{###} |
| 3 | Pimecrolimus | 22±9 | 22±6 | 44+12 |
| 4 | Compound 1 | 18+6 | 18±5* | 36±6* |
| 5 | Compound 16 | 17+4 | 16+4** | 33±6* |

| | | | | |
|---|---|---|---|---|
| Note: Compared with the blank control group, ^{##}P < 0.01, ^{###}P < 0.001; compared with the model (with matrix) group, *P < 0.05, **P < 0.01, ***P < 0.001. | | | | |

The experimental results showed that Compounds 1 and 16 of the present disclosure exhibited significant anti-pruritic effects in the chloroquine-induced mouse pruritus model and could significantly reduce the number of scratching bouts in mice (P < 0.05).

Compounds 2, 3, 9, 17, 18, and 21 of the present disclosure also exhibited anti-pruritic effects in the chloroquine-induced mouse pruritus model and could significantly reduce the number of scratching bouts in mice, as determined by the methods described above.

### Experimental Example 7: In Vitro Inhibition of ROS Release Assay

7.1 Cells: HaCaT cells

### 7.2 Reagents

Culture medium: DMEM containing 10% FBS;
Test article: Compound 16; control drugs: triethanolamine, hydrocortisone butyrate;
Reagent: ROS assay kit (Beyotime);
Instruments: RS 2000·Pro 225 Rad Source X-ray irradiator; flow cytometer.

### 7.3 Methods

### 7.3.1 Cell Culture, Grouping, and Administration

The cells were divided into a blank control group, a model group, a Compound 16 group, a triethanolamine group, and a hydrocortisone butyrate group, with 3 replicate wells per group. The cells were cultured in DMEM containing 10% FBS in a 37°C, 5% CO₂ incubator. The blank control group and the model group were treated with the corresponding volume of blank solvent (DMSO) only. The Compound 16 group was treated with 0.5 µM Compound 16. The triethanolamine group was treated with 0.5 µM triethanolamine. The hydrocortisone butyrate group was treated with 10 nM hydrocortisone butyrate.

### 7.3.2 Cell X-ray Irradiation and ROS Level Detection

At the end of administration, the cells in the model group, the Compound 16 group, the triethanolamine group, and the hydrocortisone butyrate group were exposed to 8 Gy X-ray irradiation. The blank control group received no irradiation. The intracellular ROS levels in each group were detected by flow cytometry 24 h after irradiation, according to the instructions of the ROS assay kit.

### 7.4 ROS Detection Results

| No. | Group | ROS level |
|---|---|---|
| 1 | Blank control | 1.0+0.2 |
| 2 | Model | 95.0±15.2^{##} |
| 3 | Compound 16 | 12.7±6.1*** |
| 4 | Triethanolamine | 89.0+16.5 |
| 5 | Hydrocortisone butyrate | 11.8±5.6*** |

| | | |
|---|---|---|
| Note: Compared with the blank control group, ^{###}P < 0.001; compared with the model group, ***P < 0.001. | | |

The experimental results showed that Compound 16 of the present disclosure could significantly reduce the intracellular ROS levels (P < 0.001).

Compounds 1, 2, 3, 9, 17, 18, and 21 of the present disclosure could also significantly reduce the intracellular ROS levels, as determined by the methods described above.

### Experimental Example 8: Tissue Distribution Study

8.1 Animals: ICR mice.

### 8.2 Methods

3 mg of Compound 16 was accurately weighed, added to solvent (5% DMSO + 5% Tween 80 + 20% PEG400 + 70% double-distilled water), and ultrasonicated until completely dissolved to prepare a 3 mg/mL dosing solution. ICR mice were fasted for 16 h before administration, allowed free access to food and water during the study, and administered intragastrically at a dose of 30 mg/kg. The sampling sites and time points were as follows:

| Sampling site | Pharmacokinetic time point |
|---|---|
| | Tissue: 1, 2, and 4 h after administration; |
| Plasma, liver, kidney, lung, colon, skin | plasma: 1, 2, 4, and 24 h after administration |

### 8.3 Experimental Results

| Pharmacokinetic parameters in plasma and tissues | | | | | | |
|---|---|---|---|---|---|---|
| Group | Plasma | Liver | Lung | Kidney | Colon | Skin |
| Tmax (h) | 1 | 2 | 1 | 4 | 1 | - |
| Cₘₐₓ (ng/g) | 528 | 2178 | 1137 | 1212 | 198950 | - |

The experimental results showed that the compound of the present disclosure exhibited prominent drug distribution in the colon, suggesting its potential in the treatment of colitis. Compounds 17 and 18 of the present disclosure showed similar tissue distribution profiles to Compound 16, as determined by the methods described above.

For the purpose of description and disclosure, all patents, patent applications, and other publications are expressly incorporated herein by reference. These publications are provided solely because their publication dates precede the filing date of the present application. All statements as to the dates of these documents or representations as to the contents of these documents are based on the information available to the applicant and do not constitute any admission as to the correctness of the dates or contents of these documents. Moreover, in any country, any reference herein to such publications does not constitute an admission that such publications form part of the common general knowledge in the art.

Finally, it should be noted that the above embodiments are only used to illustrate the technical solutions of the present disclosure rather than to limit them. Although the present disclosure has been described in detail with reference to the foregoing embodiments, those of ordinary skill in the art should understand that they can still make modifications to the technical solutions described in the foregoing embodiments, or make equivalent substitutions to some or all of the technical features thereof. Such modifications or substitutions do not cause the essence of the corresponding technical solutions to depart from the scope of the technical solutions of the embodiments of the present disclosure.

## Claims

1. A glycyrrhetinic acid derivative having a structure as shown in formula (II), formula (I), or formula (III), or a pharmaceutically acceptable salt thereof: 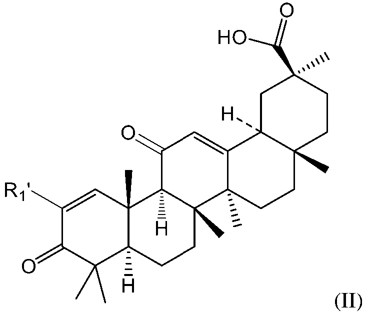
wherein, in the compound of formula (II), R₁' represents Br or Cl;
----- represents that the H atom attached to the C18-position is in an α-configuration or a β-configuration;
or, 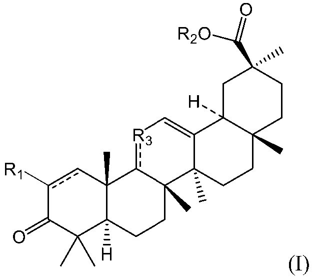
wherein, in the compound of formula (I), R₁ is selected from hydrogen, halogen, hydroxyl, cyano, methoxy, amino, or haloalkyl;
R₂ is selected from -(CH₂)ₙ-NR₂₁R₂₂, where R₂₁ and R₂₂ may be the same or different and are each independently selected from hydrogen and C1-C6 alkyl, or R₂₁ and R₂₂, together with the nitrogen atom to which they are attached, form a 5- to 6-membered heterocyclic group, which heterocyclic group may optionally contain one or more additional heteroatoms selected from O, N, or NH, and n = an integer from 1 to 6;
R₃ is selected from carbonyl or methine;
-̅ -̅ -̅ -̅ is a single bond or a double bond;
----- represents that the H atom attached to the C18-position is in the α-configuration or β-configuration;
or, 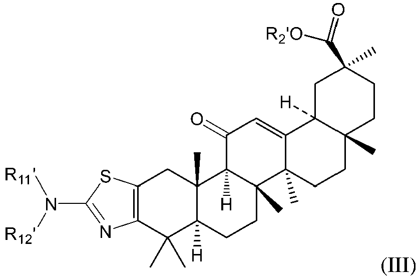
wherein, in the compound of formula (III), R₁₁' and R₁₂' are each independently selected from C1-C6 alkyl;
R₂' is selected from hydrogen or -(CH₂)ₙ-NR₂₁'R₂₂', where R₂₁' and R₂₂' may be the same or different and are each independently selected from hydrogen and C1-C6 alkyl, or R₂₁' and R₂₂', together with the nitrogen atom to which they are attached, form a 5- to 6-membered heterocyclic group, which heterocyclic group may optionally contain one or more additional heteroatoms selected from O, N, or NH, and n = an integer from 1 to 6;
----- represents that the H atom attached to the C18-position is in the α-configuration or β-configuration;
wherein the compound of formula (I) is not selected from

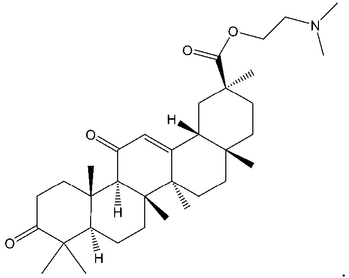

2. The glycyrrhetinic acid derivative according to claim 1, wherein, in the compound of formula (I), R₁ is selected from hydrogen or cyano;
R₂ is selected from -(CH₂)ₙ-NR₂₁R₂₂, where R₂₁ and R₂₂ may be the same or different and are each independently selected from C1-C3 alkyl, or R₂₁ and R₂₂, together with the nitrogen atom to which they are attached, form a 5- to 6-membered heterocyclic group, which heterocyclic group may optionally contain one or more additional heteroatoms selected from O, and n = an integer from 2 to 5;
R₃ is selected from carbonyl or methine;
-̅ -̅ -̅ -̅ is a single bond or a double bond;
----- represents that the H atom attached to the C18-position is in the α-configuration or β-configuration;
preferably, in the compound of formula (I), R₁ is selected from hydrogen or cyano;
R₂ is selected from -(CH₂)ₙ-NR₂₁R₂₂, where R₂₁ and R₂₂ may be the same or different and are each independently selected from methyl and ethyl, or R₂₁ and R₂₂, together with the nitrogen atom to which they are attached, form a 5- to 6-membered heterocyclic group, namely 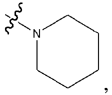 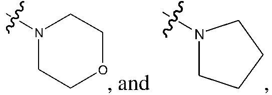 and n = an integer from 2 to 5;
R₃ is selected from carbonyl or methine;
-̅ -̅ -̅ -̅ is a single bond or a double bond;
----- represents that the H atom attached to the C18-position is in the α-configuration or β-configuration.

3. The glycyrrhetinic acid derivative according to claim 1 or 2, wherein, in the compound of formula (I), R₁ is selected from hydrogen or cyano;
R₂ is selected from -(CH₂)ₙ-NR₂₁R₂₂, where R₂₁ and R₂₂ may be the same or different and are each independently selected from methyl and ethyl, or R₂₁ and R₂₂, together with the nitrogen atom to which they are attached, form a 5- to 6-membered heterocyclic group, namely 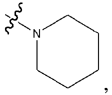 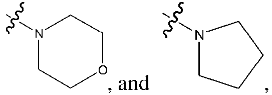 and n = an integer from 2 to 5;
R₃ is selected from carbonyl;
-̅ -̅ -̅ -̅ is a single bond or a double bond;
----- represents that the H atom attached to the C18-position is in the α-configuration or β-configuration;
preferably, in the compound of formula (I), R₁ is selected from hydrogen or cyano;
R₂ is selected from -(CH₂)ₙ-NR₂₁R₂₂, where R₂₁ and R₂₂ may be the same or different and are each independently selected from ethyl, and n = 2;
R₃ is selected from carbonyl;
-̅ -̅ -̅ -̅ is a single bond or a double bond;
----- represents that the H atom attached to the C18-position is in the α-configuration or β-configuration.

4. The glycyrrhetinic acid derivative according to any one of claims 1 to 3, wherein, in the compound of formula (I), R₁ is selected from cyano;
R₂ is selected from -(CH₂)ₙ-NR₂₁R₂₂, where R₂₁ and R₂₂ may be the same or different and are each independently selected from methyl and ethyl, and n = 2;
R₃ is selected from methine;
-̅ -̅ -̅ -̅ is a double bond;
----- represents that the H atom attached to the C18-position is in the α-configuration or β-configuration.

5. The glycyrrhetinic acid derivative according to claim 1 or 2, wherein, in the compound of formula (I), when R₁ is hydrogen, R₂ is -(CH₂)ₙ-NR₂₁R₂₂, where R₂₁ and R₂₂ may be the same or different and are each independently selected from C2-C3 alkyl, or R₂₁ and R₂₂, together with the nitrogen atom to which they are attached, form a 5- to 6-membered heterocycloalkyl group, n = an integer from 2 to 5, and the heterocycloalkyl group may optionally additionally contain one or more heteroatoms selected from O or N; R₃ is carbonyl; -̅ -̅ -̅ -̅ is a single bond; and ----- represents that the H atom attached to the C18-position is in the α-configuration or β-configuration;
or, when R₁ is cyano or amino, R₂ is -(CH₂)ₙ-NR₂₁R₂₂, where R₂₁ and R₂₂ may be the same or different and are each independently selected from C1-C3 alkyl, and n = an integer from 2 to 5; R₃ is methine or carbonyl; -̅ -̅ -̅ -̅ is a single bond or a double bond; and ----- represents that the H atom attached to the C18-position is in the α-configuration or β-configuration.

6. The glycyrrhetinic acid derivative according to any one of claims 1, 2, and 5, wherein, in the compound of formula (I), when R₁ is hydrogen, R₂ is -(CH₂)ₙ-NR₂₁R₂₂, where R₂₁ and R₂₂ may be the same or different and are each independently selected from C2-C3 alkyl, or R₂₁ and R₂₂, together with the nitrogen atom to which they are attached, form 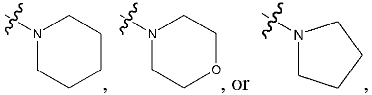 and n = an integer from 2 to 5; R₃ is carbonyl; -̅ -̅ -̅ -̅ is a single bond; and ----- represents that the H atom attached to the C18-position is in the α-configuration or β-configuration;
or, when R₁ is cyano, R₂ is -(CH₂)ₙ-NR₂₁R₂₂, where R₂₁ and R₂₂ may be the same or different and are each independently selected from C1-C3 alkyl, and n = 2 or 3; R₃ is carbonyl; -̅ -̅ -̅ -̅ is a single bond or a double bond; and ----- represents that the H atom attached to the C18-position is in the α-configuration or β-configuration.

7. The glycyrrhetinic acid derivative according to any one of claims 1, 2, 5, and 6, wherein, in the compound of formula (I), R₁ is hydrogen or cyano; R₂ is -(CH₂)ₙ-NR₂₁R₂₂, where R₂₁ and R₂₂ may be the same or different and are each independently selected from C2-C3 alkyl, and n = 2 or 3; R₃ is carbonyl; -̅ -̅ -̅ -̅ is a single bond or a double bond; and ----- represents that the H atom attached to the C18-position is in the α-configuration or β-configuration.

8. The glycyrrhetinic acid derivative according to claim 1, wherein, in the compound of formula (III), R₁₁' and R₁₂' are each independently selected from C1-C5 alkyl;
R₂' is selected from hydrogen or -(CH₂)ₙ-NR₂₁'R₂₂', where R₂₁' and R₂₂' may be the same or different and are each independently selected from C1-C5 alkyl, and n = an integer from 1 to 3;
----- represents that the H atom attached to the C18-position is in the α-configuration or β-configuration.

9. The glycyrrhetinic acid derivative according to claim 1 or 8, wherein, in the compound of formula (III), R₁₁' and R₁₂' are each independently selected from C1-C3 alkyl;
R₂' is selected from hydrogen or -(CH₂)ₙ-NR₂₁'R₂₂', where R₂₁' and R₂₂' may be the same or different and are each independently selected from C1-C3 alkyl, and n = an integer from 1 to 3;
----- represents that the H atom attached to the C18-position is in the α-configuration or β-configuration;
preferably, in the compound of formula (III), R₁₁' and R₁₂' are each independently selected from methyl, ethyl, or propyl;
R₂' is selected from hydrogen or -(CH₂)ₙ-NR₂₁'R₂₂', where R₂₁' and R₂₂' may be the same or different and are each independently selected from methyl, ethyl, or propyl, preferably ethyl, and n = 2;
----- represents that the H atom attached to the C18-position is in the α-configuration or β-configuration;
further preferably, in the compound of formula (III), R₁₁' and R₁₂' are each independently selected from methyl, ethyl, or propyl;
R₂' is selected from hydrogen;
----- represents that the H atom attached to the C18-position is in the α-configuration or β-configuration.

10. The glycyrrhetinic acid derivative according to any one of claims 1 to 7, wherein the compound of formula (I) is selected from the following compounds: 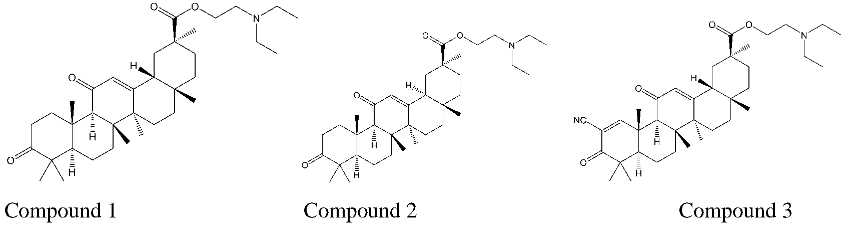 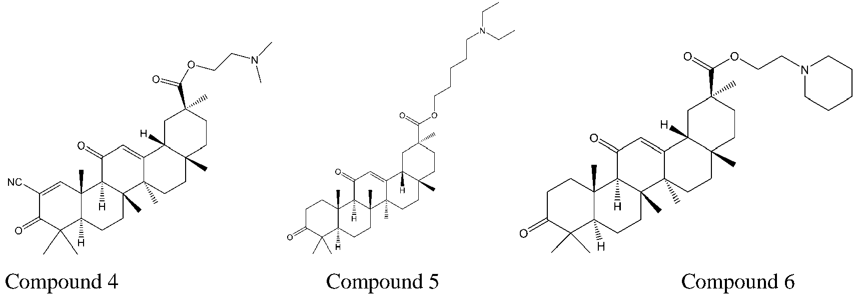 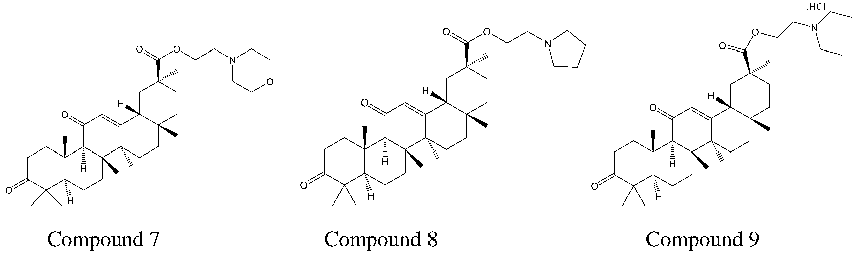 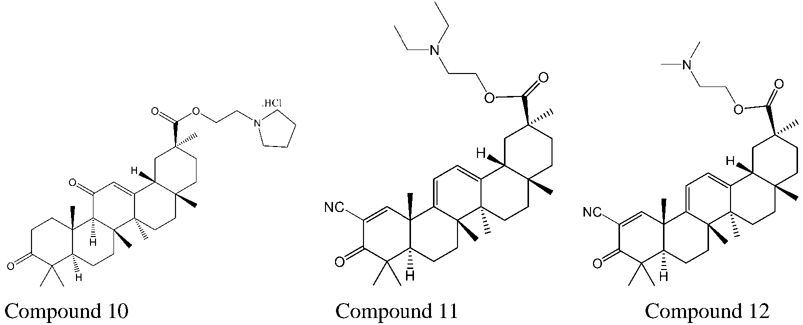 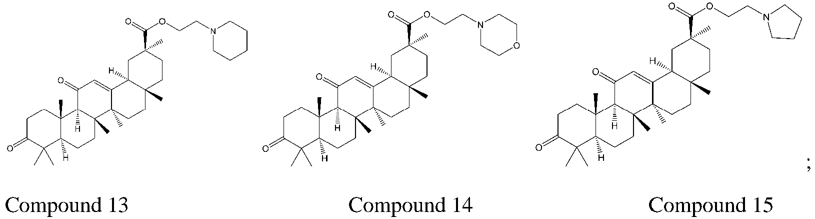 preferably Compound 1, Compound 2, Compound 3, Compound 4, Compound 5, Compound 6, Compound 7, Compound 8, Compound 9, Compound 10, Compound 13, Compound 14, and Compound 15;
further preferably Compound 1, Compound 2, Compound 3, and Compound 9.

11. The glycyrrhetinic acid derivative according to claim 1, wherein the compound of formula (II) is selected from the following compounds: 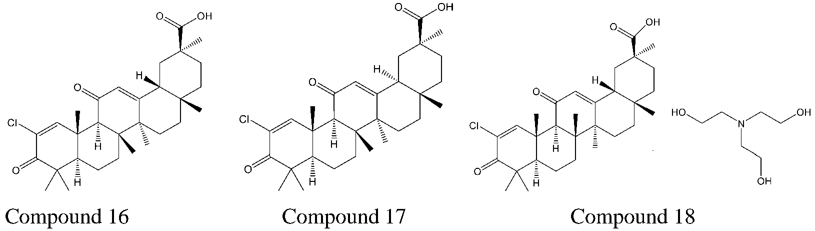 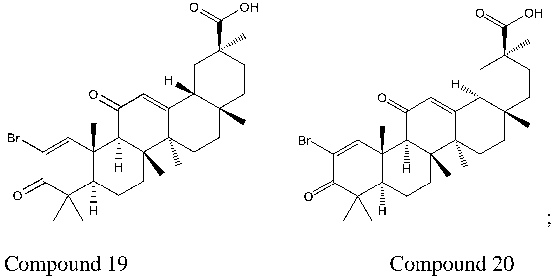 preferably Compound 16, Compound 17, and Compound 18.

12. The glycyrrhetinic acid derivative according to any one of claims 1, 8, and 9, wherein the compound of formula (III) is selected from the following compounds: 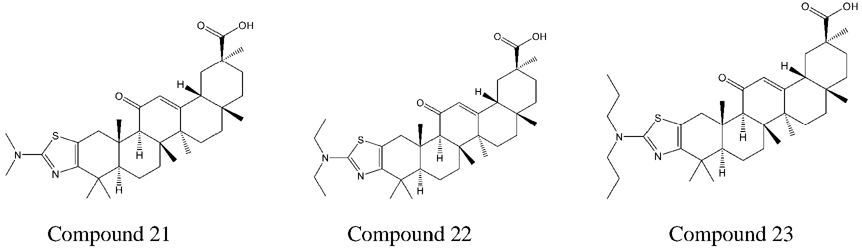 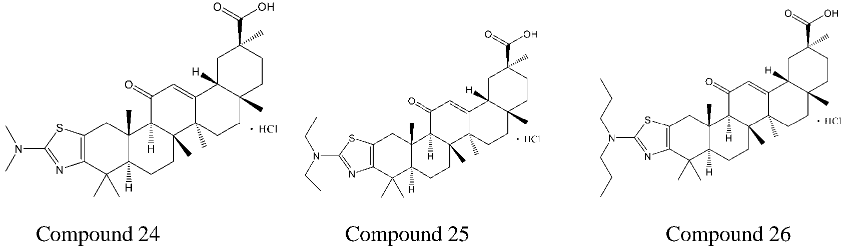 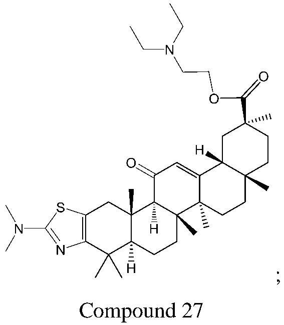 preferably Compound 21, Compound 22, and Compound 23.

13. A pharmaceutical composition, comprising the glycyrrhetinic acid derivative according to any one of claims 1 to 12, and optionally one or more pharmaceutically acceptable carriers, diluents, excipients, or adjuvants.

14. The pharmaceutical composition according to claim 13, wherein the pharmaceutical composition is a solid oral formulation, a liquid oral formulation, an injection, or a topical formulation; preferably, the solid oral formulation is selected from a tablet, capsule, dry suspension, or granule; the liquid oral formulation is selected from a syrup, oral solution, oral suspension, or oral emulsion; the injection is selected from an injection solution, sterile powder for injection, or concentrated solution for injection; the topical formulation is selected from an ointment, cream, gel, latex, paste, liniment, paint, tincture, lotion, film-forming agent, spray, suppository, foam, or enema.

15. The glycyrrhetinic acid derivative according to any one of claims 1 to 12, or the pharmaceutical composition according to claim 13 or 14, for use in the treatment of inflammatory diseases or autoimmune diseases;
preferably, the diseases are selected from dermatitis, eczema, atopic dermatitis, seborrhoeic dermatitis, hormone-dependent dermatitis, photosensitive dermatitis, chloasma, psoriasis, urticaria, prurigo nodularis, pityriasis rosea, inflammatory bowel disease, lupus erythematosus, alopecia areata, pemphigus, bullous pemphigoid, chronic nephritis, asthma, rhinitis, skin lesions caused by radiotherapy, burns, scalds, or solar dermatitis.
